# EUROPEAN PATENT APPLICATION

(11) **EP 4 843 988 A1**
(43) Date of publication of application: **25.11.2026**
(21) Application number: 25741609.9
(22) Date of filing: 17.01.2025
(51) Int. Cl.: C07D 495/04, A61K 31/4365, A61P 3/10, A61P 9/04, A61P 13/12

(54) **PYRIDOTHIOPHENE-2-CARBOXYLIC ACID DERIVATIVE, PREPARATION METHOD THEREFOR AND USE THEREOF**

(30) Priority: 18.01.2024 CN 202410075617; 05.02.2024 CN 202410162463; 15.03.2024 CN 202410298683
(71) Applicant: Tuojie Biotech (Shanghai) Co., Ltd., Shanghai 201203 (CN)
(72) Inventor: ZHU, Guodong, Shanghai 201203 (CN); LI, Yunfei, Shanghai 201203 (CN); CHEN, Dongsheng, Shanghai 201203 (CN)
(74) Representative: Regimbeau
(86) International application number: PCT/CN2025/072932
(87) International publication number: WO 2025/153045

(57) **Abstract**

The present invention relates to a pyridothiophene-2-carboxylic acid derivative, a preparation method therefor and a use thereof, and in particular to a compound as shown in formula (I) or a pharmaceutical salt thereof, which has a good inhibition/degradation effect on BCKDK. Substituents in the formula are as defined in the description.

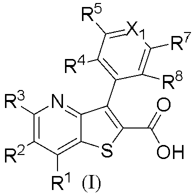

## Description

### TECHNICAL FIELD

The present disclosure relates to the field of medicinal chemistry, and in particular to a pyridothiophene-2-carboxylic acid derivative, a preparation method therefor, and use thereof.

### BACKGROUND

Branched-chain keto acid dehydrogenase kinase (BCKDK) is associated with various human diseases. In addition to regulating the catabolism of branched-chain amino acids (BCAAs), BCKDK can also enhance MEK/ERK signaling associated with malignant proliferation of cancer.

BCAAs (leucine, isoleucine, and valine) are essential amino acids in the human body, accounting for about 40% of the essential amino acids in healthy subjects. They must be obtained through a balanced diet and play an important role in nutrient sensing and cell signaling.

Branched-chain amino acids are toxic in excess, but are necessary for protein synthesis and cell signaling processes. Branched-chain amino acids are converted by the branched-chain aminotransferase (BCAT) into the α-keto acid form: α-ketoisocaproic acid (KIC/ketoleucine), 2-keto-3-methylpentanoic acid (KMV/ketoisoleucine), and α-ketoisopentanoic acid (KIV/ketovaline). Branched-chain keto acids (BCKAs) are then oxidized and decarboxylated by a branched-chain keto acid dehydrogenase (BCKDH) complex. This complex consists of multiple copies of BCKDH Ela/p tetramer, BCKDH E2, and BCKDH E3 subunits. This complex is regulated by the inhibitory phosphorylation mediated by a BCKDH kinase (BCKDK), and the same phosphorylation site is dephosphorylated by a phosphatase PPM1K. Inhibiting complex phosphorylation promotes BCKDH activity, thereby promoting irreversible catabolism of BCKAs (Lynch CJ, Adams SH: Branched-chain amino acids in metabolic signalling and insulin resistance. Nat Rev Endocrinol 2014, 10:723-36.). The absence of BCKDK in mice confirms this regulation, as BCKDK-deficient mice exhibit enhanced BCKDH activity in various tissues (Joshi MA, Jeoung NH, Obayashi M, Hattab EM, Brocken EG, Liechty EA, Kubek MJ, Vattem KM, Wek RC, Harris RA: Impaired growth and neurological abnormalities in branched-chain alpha-keto acid dehydrogenase kinase-deficient mice. Biochem J 2006, 400:153-62.).

Insufficient catabolism of branched-chain amino acids (BCAAs) is associated with insulin resistance, diabetes, congenital heart defect, and heart failure. Circulating branched-chain amino acids and their breakdown products, branched-chain α-keto acids (BCKAs), are also indicators of the onset of diabetes. The α-keto acid dehydrogenase (BCKDH) complex is a rate-limiting step in the breakdown and clearance of branched-chain amino acids; while BCKDK phosphorylates E1α subunits of BCKDH, thereby inhibiting its function.

In recent years, although there have been some early studies related to BCKDK, such as WO2020056155A, WO2020261144A, WO2020261205A, WO2022175959A, and WO2023100061A, there remains a need to develop novel and effective BCKDK inhibitors for the treatment of diseases or disorders associated with elevated BCAA concentrations.

### SUMMARY

The present disclosure provides a compound represented by formula (I) or a pharmaceutically acceptable salt thereof, 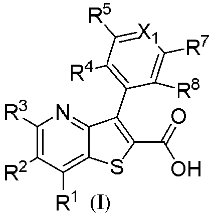
wherein R¹, R², and R³ are each independently selected from the group consisting of hydrogen, fluorine, chlorine, and methyl;
R⁴ is selected from the group consisting of fluorine and chlorine;
R⁵ is selected from the group consisting of C₁₋₄ alkyl, C₁₋₄ haloalkyl, 3- to 4-membered cycloalkyl, and -L₁-R⁹, wherein L₁ is selected from the group consisting of sulfur and oxygen, and R⁹ is selected from the group consisting of C₁₋₄ alkyl, C₁₋₄ haloalkyl, deuterated C₁₋₄ alkyl, and 3- to 4-membered cycloalkyl;
X₁ is selected from the group consisting of nitrogen and CR⁶, wherein R⁶ is selected from the group consisting of hydrogen, fluorine, and chlorine;
R⁷ and R⁸ are each independently selected from the group consisting of hydrogen, fluorine, and chlorine.

In some embodiments, in the compound represented by formula (I) or the pharmaceutically acceptable salt thereof provided in the present disclosure, X₁ is nitrogen. In some embodiments, the compound represented by formula (I) or the pharmaceutically acceptable salt thereof provided in the present disclosure is a compound represented by formula (II) or a pharmaceutically acceptable salt thereof, 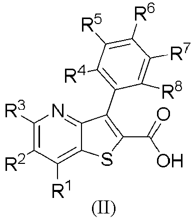 wherein R¹, R², R³, R⁴, R⁵, R⁶, R⁷, and R⁸ are each as defined in formula (I).

In some embodiments, in the compound represented by formula (I) or (II) or the pharmaceutically acceptable salt thereof provided in the present disclosure, R⁵ is selected from the group consisting of C₁₋₄ alkyl and C₁₋₄ haloalkyl.

In an optional embodiment, in the compound represented by formula (I) or (II) or the pharmaceutically acceptable salt thereof provided in the present disclosure, R⁵ is selected from the group consisting of methyl, ethyl, and isopropyl.

In some embodiments, in the compound represented by formula (I) or (II) or the pharmaceutically acceptable salt thereof provided in the present disclosure, R⁵ is selected from the group consisting of methyl and ethyl.

In some embodiments, in the compound represented by formula (I) or (II) or the pharmaceutically acceptable salt thereof provided in the present disclosure, R⁵ is selected from the group consisting of 3- to 4-membered cycloalkyl.

In an optional embodiment, in the compound represented by formula (I) or (II) or the pharmaceutically acceptable salt thereof provided in the present disclosure, R⁵ is cyclopropyl.

In some embodiments, the compound represented by formula (I) or (II) or the pharmaceutically acceptable salt thereof provided in the present disclosure is a compound represented by formula (II-1) or a pharmaceutically acceptable salt thereof, 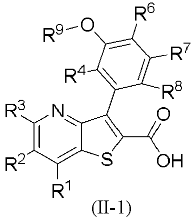 wherein R¹, R², R³, R⁴, R⁶, R⁷, R⁸, and R⁹ are each as defined in formula (I).

In some embodiments, in the compound represented by formula (I), (II), or (II-1) or the pharmaceutically acceptable salt thereof provided in the present disclosure, R⁹ is selected from the group consisting of C₁₋₄ alkyl, C₁₋₄ haloalkyl, and deuterated C₁₋₄ alkyl.

In an optional embodiment, in the compound represented by formula (I), (II), or (II-1) or the pharmaceutically acceptable salt thereof provided in the present disclosure, R⁹ is selected from the group consisting of methyl, ethyl, difluoromethyl, trifluoromethyl, and deuterated methyl.

In an optional embodiment, in the compound represented by formula (I), (II), or (II-1) or the pharmaceutically acceptable salt thereof provided in the present disclosure, R⁹ is selected from the group consisting of methyl, difluoromethyl, trifluoromethyl, and deuterated methyl.

In some embodiments, the compound represented by formula (I) or (II) or the pharmaceutically acceptable salt thereof provided in the present disclosure is a compound represented by formula (II-2) or a pharmaceutically acceptable salt thereof, 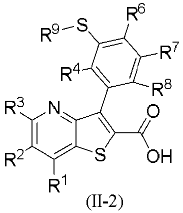 wherein R¹, R², R³, R⁴, R⁶, R⁷, R⁸, and R⁹ are each as defined in formula (I).

In some embodiments, in the compound represented by formula (II-2) or the pharmaceutically acceptable salt thereof provided in the present disclosure, R⁹ is selected from the group consisting of C₁₋₄ alkyl, C₁₋₄ haloalkyl, and deuterated C₁₋₄ alkyl.

In an optional embodiment, in the compound represented by formula (II-2) or the pharmaceutically acceptable salt thereof provided in the present disclosure, R⁹ is selected from the group consisting of methyl, ethyl, difluoromethyl, trifluoromethyl, and deuterated methyl.

In an optional embodiment, in the compound represented by formula (II-2) or the pharmaceutically acceptable salt thereof provided in the present disclosure, R⁹ is methyl. In some embodiments, in the compound represented by formula (I), (II), (II-1), or (II-2) or the pharmaceutically acceptable salt thereof provided in the present disclosure, at least one of R¹, R², and R³ is selected from the group consisting of fluorine and chlorine.

In some embodiments, in the compound represented by formula (I), (II), (II-1), or (II-2) or the pharmaceutically acceptable salt thereof provided in the present disclosure, R¹ and R³ are each independently selected from the group consisting of hydrogen, fluorine, and chlorine.

In an optional embodiment, in the compound represented by formula (I), (II), (II-1), or (II-2) or the pharmaceutically acceptable salt thereof provided in the present disclosure, one of R¹ and R³ is hydrogen, and the other is selected from the group consisting of hydrogen, fluorine, and chlorine.

In an optional embodiment, in the compound represented by formula (I), (II), (II-1), or (II-2) or the pharmaceutically acceptable salt thereof provided in the present disclosure, R¹ and R³ are each independently hydrogen.

In some embodiments, in the compound represented by formula (I), (II), (II-1), or (II-2) or the pharmaceutically acceptable salt thereof provided in the present disclosure, R² is fluorine or chlorine.

In some embodiments, in the compound represented by formula (I), (II), (II-1), or (II-2) or the pharmaceutically acceptable salt thereof provided in the present disclosure, R² is fluorine.

In some embodiments, in the compound represented by formula (I), (II), (II-1), or (II-2) or the pharmaceutically acceptable salt thereof provided in the present disclosure, R² is chlorine.

In some embodiments, in the compound represented by formula (I), (II), (II-1), or (II-2) or the pharmaceutically acceptable salt thereof provided in the present disclosure, R¹ and R³ are each independently hydrogen; R² is fluorine or chlorine.

In some embodiments, in the compound represented by formula (I), (II), (II-1), or (II-2) or the pharmaceutically acceptable salt thereof provided in the present disclosure, R⁴ is selected from the group consisting of fluorine and chlorine.

In some embodiments, in the compound represented by formula (I), (II), (II-1), or (II-2) or the pharmaceutically acceptable salt thereof provided in the present disclosure, R⁴ is fluorine.

In some embodiments, in the compound represented by formula (I), (II), (II-1), or (II-2) or the pharmaceutically acceptable salt thereof provided in the present disclosure, R¹ and R³ are each independently hydrogen, R² is fluorine or chlorine, and R⁴ is fluorine.

In some embodiments, in the compound represented by formula (I), (II), (II-1), or (II-2) or the pharmaceutically acceptable salt thereof provided in the present disclosure, at least one of R⁶, R⁷, and R⁸ is selected from the group consisting of fluorine and chlorine.

In an optional embodiment, in the compound represented by formula (I), (II), (II-1), or (II-2) or the pharmaceutically acceptable salt thereof provided in the present disclosure, at least two of R⁶, R⁷, and R⁸ are selected from the group consisting of fluorine and chlorine.

In some embodiments, in the compound represented by formula (I), (II), (II-1), or (II-2) or the pharmaceutically acceptable salt thereof provided in the present disclosure, R⁶ is selected from the group consisting of fluorine and chlorine.

In some embodiments, in the compound represented by formula (I), (II), (II-1), or (II-2) or the pharmaceutically acceptable salt thereof provided in the present disclosure, at least one of R⁶, R⁷, and R⁸ is chlorine.

In some embodiments, in the compound represented by formula (I), (II), (II-1), or (II-2) or the pharmaceutically acceptable salt thereof provided in the present disclosure, R⁷ is selected from the group consisting of fluorine and chlorine, and R⁸ is hydrogen.

In an optional embodiment, in the compound represented by formula (I), (II), (II-1), or (II-2) or the pharmaceutically acceptable salt thereof provided in the present disclosure, R⁷ is fluorine, and R⁸ is hydrogen.

In some embodiments, in the compound represented by formula (I), (II), (II-1), or (II-2) or the pharmaceutically acceptable salt thereof provided in the present disclosure, R⁸ is selected from the group consisting of fluorine and chlorine, and R⁷ is hydrogen.

In some embodiments, in the compound represented by formula (I), (II), (II-1), or (II-2) or the pharmaceutically acceptable salt thereof provided in the present disclosure, R¹ and R³ are each independently hydrogen; R² is fluorine or chlorine; R⁴ is fluorine; R⁶ is selected from the group consisting of fluorine and chlorine; R⁷ is selected from the group consisting of fluorine and chlorine, and R⁸ is hydrogen; or R⁸ is selected from the group consisting of fluorine and chlorine, and R⁷ is hydrogen; and at least one of R⁶, R⁷, and R⁸ is chlorine.

In some embodiments, the compound represented by formula (I), (II), (II-1), or (II-2) or the pharmaceutically acceptable salt thereof provided in the present disclosure is a compound represented by formula (II-A) or a pharmaceutically acceptable salt thereof, 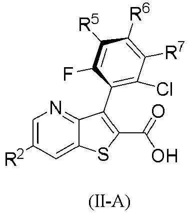 wherein R², R⁵, R⁶, and R⁷ are each as defined in formulas (I), (II), (II-1), and (II-2).

In an optional embodiment, in the compound represented by formula (II-A) or the pharmaceutically acceptable salt thereof provided in the present disclosure, R² is chlorine. In an optional embodiment, in the compound represented by formula (II-A) or the pharmaceutically acceptable salt thereof provided in the present disclosure, R² is fluorine. In some embodiments, the compound represented by formula (I), (II), (II-1), or (II-2) or the pharmaceutically acceptable salt thereof provided in the present disclosure is a compound represented by formula (II-B) or a pharmaceutically acceptable salt thereof, 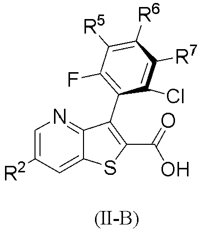 wherein R², R³, R⁶, and R⁷ are each as defined in formulas (I), (II), (II-1), and (II-2).

In an optional embodiment, in the compound represented by formula (II-B) or the pharmaceutically acceptable salt thereof provided in the present disclosure, R² is chlorine. In an optional embodiment, in the compound represented by formula (II-B) or the pharmaceutically acceptable salt thereof provided in the present disclosure, R² is fluorine. In some embodiments, in the compound represented by formula (II-A) or (II-B) or the pharmaceutically acceptable salt thereof provided in the present disclosure, R⁵ is selected from the group consisting of methyl and ethyl.

In some embodiments, in the compound represented by formula (II-A) or (II-B) or the pharmaceutically acceptable salt thereof provided in the present disclosure, R⁵ is selected from the group consisting of -L₁-R⁹, wherein L₁ is oxygen, and R⁹ is selected from the group consisting of methyl, ethyl, difluoromethyl, trifluoromethyl, and deuterated methyl. In some embodiments, in the compound represented by formula (II-A) or (II-B) or the pharmaceutically acceptable salt thereof provided in the present disclosure, R⁵ is selected from the group consisting of -L₁-R⁹, wherein L₁ is oxygen, and R⁹ is selected from the group consisting of methyl and deuterated methyl.

In some embodiments, in the compound represented by formula (II-A) or (II-B) or the pharmaceutically acceptable salt thereof provided in the present disclosure, R⁵ is selected from the group consisting of -L₁-R⁹, wherein L₁ is oxygen, and R⁹ is selected from the group consisting of difluoromethyl and trifluoromethyl.

In some embodiments, in the compound represented by formula (II-A) or (II-B) or the pharmaceutically acceptable salt thereof provided in the present disclosure, R⁵ is selected from the group consisting of -L₁-R⁹, wherein L₁ is sulfur, and R⁹ is selected from the group consisting of methyl, ethyl, difluoromethyl, trifluoromethyl, and deuterated methyl. In some embodiments, in the compound represented by formula (II-A) or (II-B) or the pharmaceutically acceptable salt thereof provided in the present disclosure, R⁵ is selected from the group consisting of -L₁-R⁹, wherein L₁ is sulfur, and R⁹ is methyl or deuterated methyl.

In some embodiments, in the compound represented by formula (II-A) or (II-B) or the pharmaceutically acceptable salt thereof provided in the present disclosure, R⁶ is fluorine or chlorine, and R⁷ is hydrogen.

In an optional embodiment, in the compound represented by formula (II-A) or (II-B) or the pharmaceutically acceptable salt thereof provided in the present disclosure, R⁶ is fluorine, and R⁷ is hydrogen.

In some embodiments, the compound represented by formula (I), (II), (II-1), or (II-2) or the pharmaceutically acceptable salt thereof provided in the present disclosure is a compound represented by formula (II-C) or a pharmaceutically acceptable salt thereof, 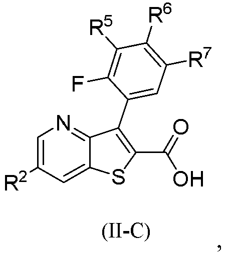 wherein R², R⁵, R⁶, and R⁷ are each as defined in formulas (I), (II), (II-1), and (II-2).

In an optional embodiment, in the compound represented by formula (II-C) or the pharmaceutically acceptable salt thereof provided in the present disclosure, R² is chlorine. In an optional embodiment, in the compound represented by formula (II-C) or the pharmaceutically acceptable salt thereof provided in the present disclosure, R² is fluorine. In some embodiments, in the compound represented by formula (II-C) or the pharmaceutically acceptable salt thereof provided in the present disclosure, R⁵ is selected from the group consisting of methyl and ethyl.

In some embodiments, in the compound represented by formula (II-C) or the pharmaceutically acceptable salt thereof provided in the present disclosure, R⁵ is selected from the group consisting of -L₁-R⁹, wherein L₁ is oxygen, and R⁹ is selected from the group consisting of methyl, ethyl, difluoromethyl, trifluoromethyl, and deuterated methyl.

In some embodiments, in the compound represented by formula (II-C) or the pharmaceutically acceptable salt thereof provided in the present disclosure, R⁵ is selected from the group consisting of -L₁-R⁹, wherein L₁ is oxygen, and R⁹ is selected from the group consisting of methyl and deuterated methyl.

In some embodiments, in the compound represented by formula (II-C) or the pharmaceutically acceptable salt thereof provided in the present disclosure, R⁵ is selected from the group consisting of -L₁-R⁹, wherein L₁ is oxygen, and R⁹ is selected from the group consisting of difluoromethyl and trifluoromethyl.

In some embodiments, in the compound represented by formula (II-C) or the pharmaceutically acceptable salt thereof provided in the present disclosure, R⁵ is selected from the group consisting of -L₁-R⁹, wherein L₁ is sulfur, and R⁹ is selected from the group consisting of methyl, ethyl, difluoromethyl, trifluoromethyl, and deuterated methyl. In some embodiments, in the compound represented by formula (II-C) or the pharmaceutically acceptable salt thereof provided in the present disclosure, R⁵ is selected from the group consisting of -L₁-R⁹, wherein L₁ is sulfur, and R⁹ is methyl or deuterated methyl.

In some embodiments, in the compound represented by formula (II-C) or the pharmaceutically acceptable salt thereof provided in the present disclosure, at least one of R⁶ and R⁷ is selected from the group consisting of fluorine and chlorine.

In an optional embodiment, in the compound represented by formula (II-C) or the pharmaceutically acceptable salt thereof provided in the present disclosure, R⁶ is selected from the group consisting of fluorine and chlorine.

In an optional embodiment, in the compound represented by formula (II-C) or the pharmaceutically acceptable salt thereof provided in the present disclosure, R⁶ is selected from the group consisting of fluorine, and R⁷ is selected from the group consisting of fluorine and chlorine.

In an optional embodiment, in the compound represented by formula (II-C) or the pharmaceutically acceptable salt thereof provided in the present disclosure, R⁶ is selected from the group consisting of chlorine, and R⁷ is selected from the group consisting of fluorine and chlorine.

The present disclosure further provides a compound or a pharmaceutically acceptable salt thereof selected from the group consisting of the following: 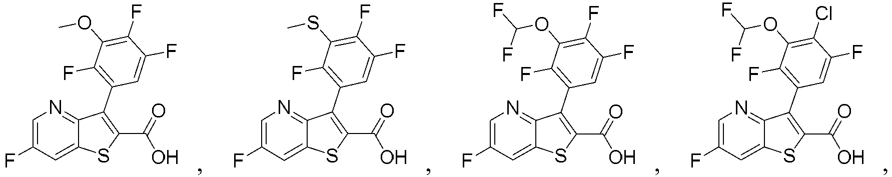 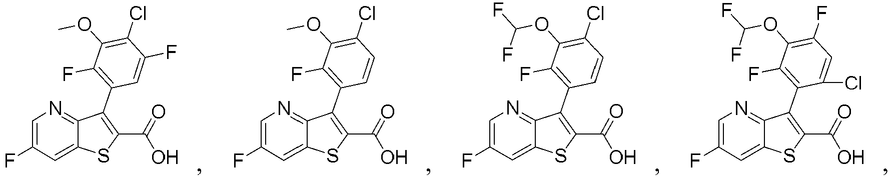 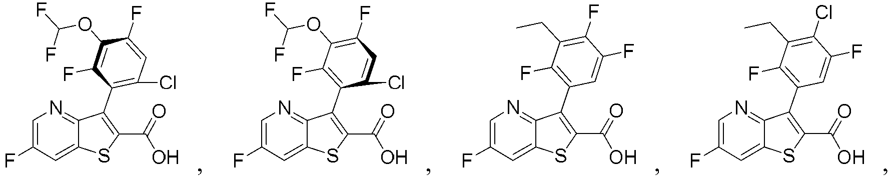 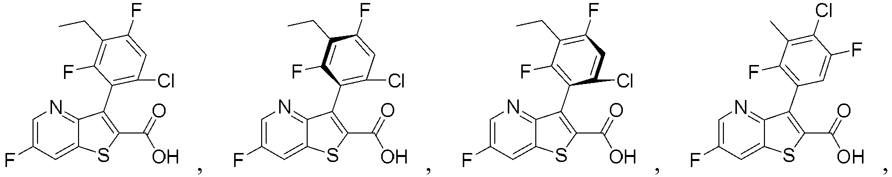 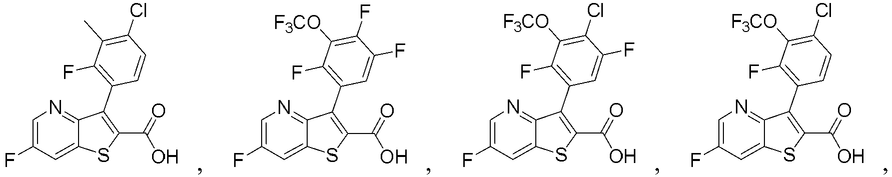 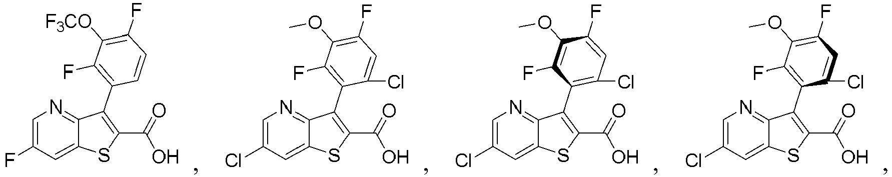 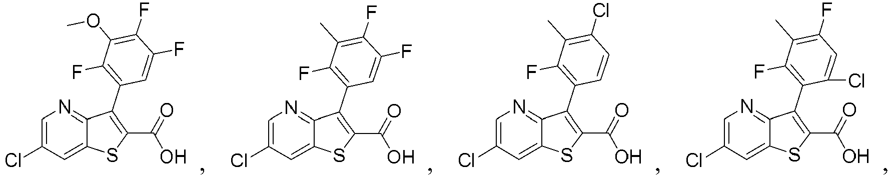 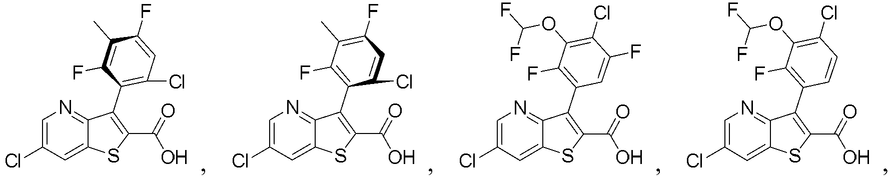 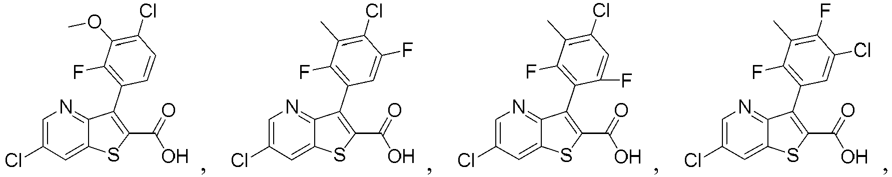 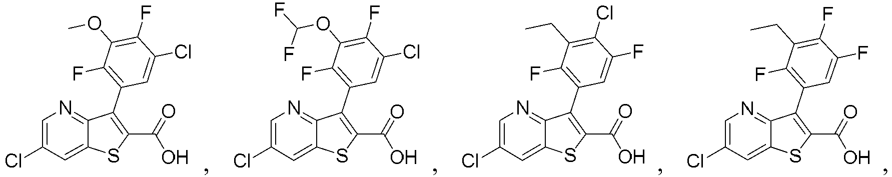 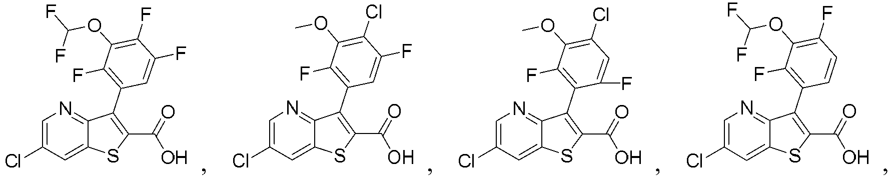 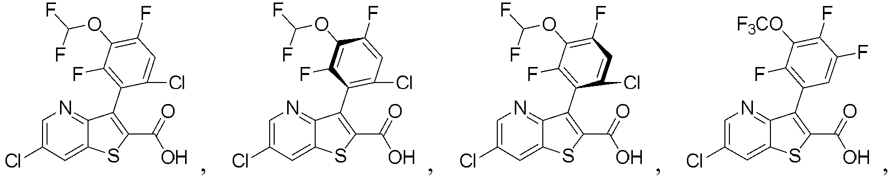 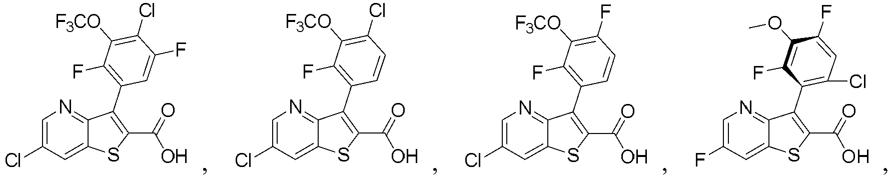 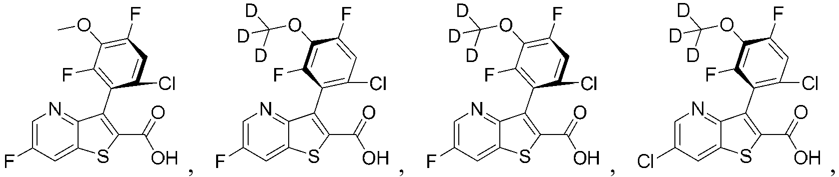 and

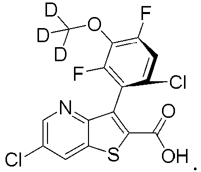

The present disclosure further provides an isotopically substituted form of the aforementioned compound or the pharmaceutically acceptable salt thereof; optionally, the isotopically substituted form is a deuterated form.

The present disclosure provides a pharmaceutical composition comprising the compound described above, and one or more pharmaceutically acceptable excipients.

In some embodiments, a unit dose of the pharmaceutical composition is 0.001 mg-1000 mg.

In certain embodiments, the pharmaceutical composition comprises 0.01%-99.99% of the aforementioned compound or the pharmaceutically acceptable salt thereof or the isotopically substituted form thereof based on the total weight of the composition. In certain embodiments, the pharmaceutical composition comprises 0.1%-99.9% of the aforementioned compound or the pharmaceutically acceptable salt thereof or the isotopically substituted form thereof. In certain embodiments, the pharmaceutical composition comprises 0.5%-99.5% of the aforementioned compound or the pharmaceutically acceptable salt thereof or the isotopically substituted form thereof. In certain embodiments, the pharmaceutical composition comprises 1%-99% of the aforementioned compound or the pharmaceutically acceptable salt thereof or the isotopically substituted form thereof. In certain embodiments, the pharmaceutical composition comprises 2%-98% of the aforementioned compound or the pharmaceutically acceptable salt thereof or the isotopically substituted form thereof.

In certain embodiments, the pharmaceutical composition comprises 0.01%-99.99% of the pharmaceutically acceptable excipient based on the total weight of the composition. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

The present disclosure further provides use of the aforementioned compound or the pharmaceutically acceptable salt thereof or the isotopically substituted form thereof or the aforementioned pharmaceutical composition as a medicament.

The present disclosure further provides use of the aforementioned compound or the pharmaceutically acceptable salt thereof or the isotopically substituted form thereof or the aforementioned pharmaceutical composition in the manufacture of a medicament for preventing and/or treating a BCKDK-related disease.

The present disclosure further provides use of the aforementioned compound or the pharmaceutically acceptable salt thereof or the isotopically substituted form thereof or the aforementioned pharmaceutical composition in the manufacture of a medicament for preventing and/or treating a disease, wherein the disease includes, but is not limited to, a glucose metabolism disorder or an abnormal blood glucose disease, a heart disease, or a kidney disease.

The present disclosure further provides a method for preventing and/or treating a BCKDK-related disease, comprising administering to a patient the aforementioned compound or the pharmaceutically acceptable salt thereof or the isotopically substituted form thereof or the aforementioned pharmaceutical composition.

The present disclosure further provides a method for preventing and/or treating a disease, comprising administering to a patient the aforementioned compound or the pharmaceutically acceptable salt thereof or the isotopically substituted form thereof or the aforementioned pharmaceutical composition, wherein the disease includes, but is not limited to, a glucose metabolism disorder or an abnormal blood glucose disease, a heart disease, or a kidney disease.

In an optional embodiment, in the method provided in the present disclosure, the aforementioned compound or the pharmaceutically acceptable salt thereof or the isotopically substituted form thereof or the aforementioned pharmaceutical composition is administered to a patient in a therapeutically effective amount.

In some embodiments, the BCKDK-related disease includes, but is not limited to, a glucose metabolism disorder or an abnormal blood glucose disease, a heart disease, or a kidney disease.

In some embodiments, the glucose metabolism disorder or the abnormal blood glucose disease is diabetes.

In some embodiments, the heart disease is heart failure.

The aforementioned compound or the pharmaceutically acceptable salt thereof or the isotopically substituted form thereof or the aforementioned pharmaceutical composition provided in the present disclosure exhibits a good inhibition/degradation effect on BCKDK.

In another aspect, the present disclosure provides a preparation method for a compound represented by formula (I) or a pharmaceutically acceptable salt thereof, comprising a step of removing a protecting group PG from a compound represented by formula (I-a) or a pharmaceutically acceptable salt thereof under an alkaline condition, and further comprising a step of adjusting the pH to acidity, 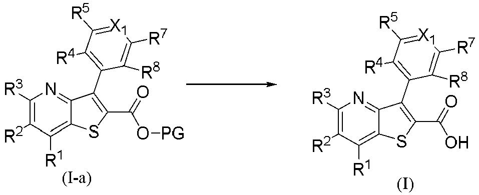 wherein PG is a carboxyl protecting group; R¹, R², R³, R⁴, R⁵, X₁, R⁷, and R⁸ are each as defined in formula (I).

In an optional embodiment, in the preparation method for the compound represented by formula (I) or the pharmaceutically acceptable salt thereof provided in the present disclosure, PG is C₁₋₆ alkyl.

In another aspect, the present disclosure provides a compound represented by formula (I-a) or a pharmaceutically acceptable salt thereof, 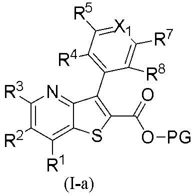 wherein PG is a carboxyl protecting group; R¹, R², R³, R⁴, R⁵, X₁, R⁷, and R⁸ are each as defined in formula (I).

In an optional embodiment, in the compound represented by formula (I-a) or the pharmaceutically acceptable salt thereof, PG is C₁₋₆ alkyl.

### Definitions of Terms

Where the present disclosure does not define a particular configuration, the compounds of the present disclosure may exist in particular geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, atropisomers (i.e., stereoisomers with hindered rotation), and racemic mixtures and other mixtures thereof, such as enantiomerically or diastereomerically enriched mixtures, all of which fall within the scope of the present disclosure. Additional asymmetric carbon atoms may be present in substituents such as an alkyl group. All such isomers and mixtures thereof are included within the scope of the present disclosure.

In addition, the compounds and intermediates of the present disclosure may also exist in different tautomeric forms, and all such forms are included within the scope of the present disclosure. The term "tautomer" or "tautomeric form" refers to structural isomers of different energies that can interconvert via a low-energy barrier.

The compounds of the present disclosure may be asymmetric, for example, having one or more stereoisomers. Unless otherwise specified, all stereoisomers are included, for example, enantiomers and diastereomers. The compounds of the present disclosure containing asymmetric carbon atoms may be separated in an optically active pure form or in a racemic form. The optically active pure form may be isolated from a racemic mixture or synthesized using chiral starting materials or chiral reagents.

Optically active (R)- and (S)-isomers, and D- and L-isomers may be prepared by chiral synthesis, chiral reagents, or other conventional techniques. If one enantiomer of a certain compound of the present disclosure is desired, it may be prepared by asymmetric synthesis or derivatization with a chiral auxiliary, wherein the resulting mixture of diastereomers is separated, and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, diastereomeric resolution is then performed by conventional methods well-known in the art, and pure enantiomers are then recovered. In addition, the separation of enantiomers and diastereomers is generally accomplished by chromatography using a chiral stationary phase, optionally in combination with chemical derivatization (e.g., formation of a carbamate from an amine). In the chemical structures of the compounds of the present disclosure, the bond " 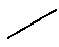 " indicates an unspecified configuration; that is, if chiral isomers exist in the chemical structures, the bond " 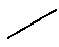 " may be " 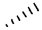 " or " 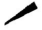 ", or includes both the configurations " 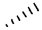 " and " 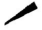 " simultaneously. In the chemical structures of the compounds of the present disclosure, the bond " 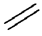 " does not specify a configuration; that is, the configuration of the bond " 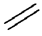 " may be an *E* configuration or a *Z* configuration, or includes both the *E* configuration and the *Z* configuration simultaneously.

The present disclosure also includes some isotopically labeled compounds of the present disclosure that are identical to those recited herein but have one or more atoms replaced with an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Unless otherwise specified, when a position is specifically designated as deuterium (D), the position shall be understood to be deuterium having an abundance that is at least 1000 times greater than the natural abundance of deuterium (which is 0.015%) (i.e., at least 10% deuterium incorporation). The compounds in examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure also includes various deuterated forms of the compounds. Each available hydrogen atom linked to a carbon atom may be independently replaced with a deuterium atom. Those skilled in the art can synthesize the deuterated forms of the compounds by referring to the relevant literature. Commercially available deuterated starting materials can be used in preparing the deuterated forms of the compounds, or they can be synthesized using conventional techniques with deuterated reagents, including but not limited to deuterated borane, borane-d₃ in tetrahydrofuran, deuterated lithium aluminum hydride, deuterated iodoethane, deuterated iodomethane, and the like.

The term "alkyl" refers to a saturated aliphatic hydrocarbon group, preferably an alkyl group containing 1 to 6 carbon atoms (e.g., 1, 2, 3, 4, 5, or 6 carbon atoms). Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, and 2,3-dimethylbutyl.

The term "cycloalkyl" or "carbocycle" refers to a saturated or partially unsaturated, monocyclic or polycyclic hydrocarbon substituent, and the cycloalkyl ring contains 3 to 20 carbon atoms, preferably 3 to 6 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, etc. Polycyclic cycloalkyl groups include spiro-ring, fused-ring, and bridged-ring cycloalkyl groups.

The term "alkoxy" refers to -O-(alkyl) and -O-(unsubstituted cycloalkyl), wherein the alkyl and cycloalkyl are as defined above. Non-limiting examples of alkoxy groups include methoxy, ethoxy, propoxy, butoxy, cyclopropyloxy, cyclobutoxy, cyclopentyloxy, and cyclohexyloxy.

The term "hydroxy" refers to -OH.

The term "halogen" refers to fluorine, chlorine, bromine, or iodine.

The term "haloalkyl" refers to an alkyl group substituted with halogen, wherein the alkyl group is as defined above.

The term "cyano" refers to -CN.

The term "amino" refers to -NH₂.

"Optionally" or "optional" means that the event or circumstance subsequently described may, but does not necessarily, occur; this description includes an instance where the event or circumstance occurs or does not occur. For example, "C₁₋₆ alkyl optionally substituted with halogen or cyano" means that the halogen or cyano may, but does not necessarily, exist; this description includes an instance where the alkyl is substituted with halogen or cyano and an instance where the alkyl is not substituted with halogen or cyano.

The term "substituted" means that one or more, preferably up to 5, and more preferably 1 to 3, hydrogen atoms in the group are independently substituted with a corresponding number of substituents. It goes without saying that a substituent is only in its possible chemical position, and those skilled in the art can determine (experimentally or theoretically) possible or impossible substitution without undue effort.

"Substituted with one or more..." refers to substitution with a single substituent or multiple substituents. In the case of substitution with multiple substituents, there may be a plurality of identical substituents, or one or a group of a plurality of different substituents. In the present disclosure, the terms "comprise" and "include" can be replaced with "consist of".

The term "composition" refers to a mixture of a drug containing one or more of the compounds described herein or physiologically and pharmaceutically acceptable salts or precursors thereof, and other chemical components, as well as other components such as physiologically and pharmaceutically acceptable carriers and excipients. The composition is intended to promote administration to an organism and facilitate the absorption of the active ingredient, thereby exerting biological activity.

The term "pharmaceutically acceptable excipient" includes, but is not limited to, any auxiliary, carrier, excipient, glidant, sweetener, diluent, preservative, dye/colorant, flavoring agent, surfactant, wetting agent, dispersant, suspending agent, stabilizer, isotonic agent, solvent, or emulsifier that has been approved by the U.S. Food and Drug Administration as acceptable for use in humans or livestock animals.

Unless otherwise specified, the "compound" of the present disclosure can be present in the form of a salt or mixed salt or in a non-salt form (e.g., a free acid or free base). When present in the form of a salt or mixed salt, it may be a pharmaceutically acceptable salt. The term "pharmaceutically acceptable salt" includes pharmaceutically acceptable acid addition salts and pharmaceutically acceptable base addition salts.

As used herein, the term "inhibit" is used interchangeably with "decrease", "silence", "down-regulate", "repress", and other similar terms, and includes any level of inhibition. Inhibition can be assessed in terms of a decrease in the absolute or relative level of one or more of these variables relative to a control level. The control level may be any type of control level used in the art, such as a pre-dose baseline level or a level determined from a similar untreated or control (e.g., buffer-only control or inert agent control) treated subject, cell, or sample.

"Effective amount", "effective dose", "effective therapeutic amount", or "therapeutically effective amount" refers to the amount of a drug, a compound, or a pharmaceutical composition necessary to obtain any one or more beneficial or desired therapeutic results. For prophylactic use, the beneficial or desired results include elimination or reduction of risk, reduction of severity, or delay of the onset of a disorder, including the biochemistry, histology, and/or behavioral symptoms of the disorder, complications thereof, and intermediate pathological phenotypes that appear during the progression of the disorder. As used herein, "object", "patient", "subject", and "individual" are used interchangeably and include human or non-human animals, e.g., mammals, e.g., humans or monkeys.

### DETAILED DESCRIPTION

The present disclosure is further described below with reference to examples; however, these examples are not intended to limit the scope of the present disclosure. Experimental methods without specific conditions indicated in the examples of the present disclosure were generally conducted under conventional conditions, or conditions recommended by the manufacturers of the starting materials or commercial products. Where the specific source of a reagent is not indicated, the reagent may be obtained from any supplier of molecular biology reagents at a quality/purity level for molecular biology applications.

Unless otherwise specified, all reagents used in the following examples are commercially available.

Analysis conditions used for all mass spectrometry data in the following examples: column model: ACQUITY UPLC BEH C18 1.7 µM, 2.1 × 50 mm Column; mobile phase A: 0.05% ammonia water + 0.01% formic acid in water; mobile phase B: 0.01% formic acid in acetonitrile; the gradient of mobile phase B in 0-1.7 min was 5%-95%; the gradient of mobile phase B was maintained at 95% in 1.7-2.5 min.

In the following examples, the instrument used for all proton nuclear magnetic resonance spectroscopic data was a Bruker 400 MHz nuclear magnetic resonance spectrometer; the instrument used for all fluorine nuclear magnetic resonance spectroscopic data was a Bruker 376 MHz nuclear magnetic resonance spectrometer.

### Example 1

### 6-Chloro-3-(2,4,5-trifluoro-3-methoxyphenyl)thieno[3,2-b]pyridine-2-carboxylic acid (1)

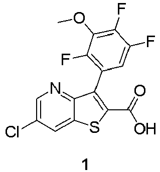

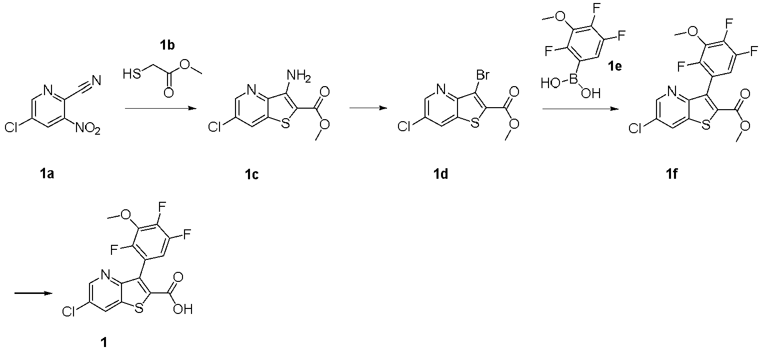

### Step 1: synthesis of methyl 3-amino-6-chlorothieno[3,2-b]pyridine-2-carboxylate 1c

At 0 °C, methyl 2-mercaptoacetate **1b** (2.4 mL, 27.24 mmol) was slowly added dropwise to a solution of 5-chloro-3-nitropyridin-2-carbonitrile **1a** (5.00 g, 27.24 mmol) in N,N-dimethylformamide (60 mL), and then a solution of potassium hydroxide (3.06 g, 54.50 mmol) in water (10.0 mL) was added dropwise. The reaction mixture was stirred at 0-5 °C for 1 h. Ice water (60 mL) was added, followed by extraction with ethyl acetate (2 × 100 mL). The organic phases were combined, washed with saturated brine (4 × 80 mL), dried over anhydrous sodium sulfate, filtered, and concentrated under reduced pressure to give a crude product of **1c** (6.5 g, yield: 98%).

MS(ESI) m/z = 243.2 [M+H]⁺.

### Step 2: synthesis of methyl 3-bromo-6-chlorothieno[3,2-b]pyridine-2-carboxylate 1d

At 25 °C, in a nitrogen atmosphere, tert-butyl nitrite (3.92 g, 1.3 mL, 10.7 mmol) was added dropwise to a three-necked flask containing copper (II) bromide (2.05 g, 9.13 mmol) and acetonitrile (15.0 mL). A suspension of compound **1c** (2.0 g, 8.3 mmol) in acetonitrile (5.0 mL) was then added at 20 °C. The reaction mixture was stirred at 25 °C for 2 h, then slowly poured into a hydrochloric acid solution (2 N, 20 mL), and extracted with ethyl acetate (2 × 70 mL). The organic phases were combined, washed with saturated brine (2 × 100 mL), dried over anhydrous sodium sulfate, filtered, and concentrated under reduced pressure to give a crude product, which was purified by column chromatography (petroleum ether/ethyl acetate) to give the title compound **1d** (1.0 g, yield: 39%).

MS(ESI) m/z = 306.1 [M+H]⁺.

### Step 3: synthesis of methyl 6-chloro-3-(2,4,5-trifluoro-3-methoxyphenyl)thieno[3,2-b]pyridine-2-carboxylate 1f

At room temperature, compound **1d** (75 mg, 0.25 mmol) and compound **1e** (50 mg, 0.25 mmol) were dissolved in 1,4-dioxane (2 mL) and water (0.4 mL), and 1,1'-di-tert-butylphosphinoferrocene palladium(II) dichloride (15.9 mg, 0.024 mmol) and potassium carbonate (101 mg, 0.73 mmol) were added. The reaction mixture was stirred at 100 °C for 1 h in a nitrogen atmosphere. After the mixture was cooled to room temperature, ethyl acetate (60 mL) was added. The mixture was washed with saturated brine (2 × 20 mL), dried over anhydrous sodium sulfate, filtered, and concentrated under reduced pressure to give a crude product, which was purified by column chromatography (petroleum ether/ethyl acetate) to give the title compound **1f** (60 mg, yield: 61%).

MS(ESI) m/z = 388.2 [M+H]⁺.

### Step 4: synthesis of 6-chloro-3-(2,4,5-trifluoro-3-methoxyphenyl)thieno[3,2-b]pyridine-2-carboxylic acid 1

At room temperature, compound **1f** (60 mg, 0.16 mmol) was dissolved in a mixed solvent of tetrahydrofuran (1 mL), methanol (0.5 mL), and water (0.5 mL), and lithium hydroxide (37 mg, 1.6 mmol) was added. The reaction mixture was stirred at room temperature for 1 h, and water (30 mL) was added. The pH was adjusted to 4-5 with hydrochloric acid (1 N), followed by extraction with ethyl acetate (2 × 10 mL). The organic phases were combined, washed with saturated brine (2 × 10 mL), dried over anhydrous sodium sulfate, filtered, and concentrated under reduced pressure to give a crude product, which was separated and purified by preparative high performance liquid chromatography (column: Waters Xbridge, 30 × 250 mm; mobile phase A: 0.1% formic acid in water; mobile phase B: acetonitrile; gradient: 40%-95%, 18 min; flow rate: 30 mL/min; peak time: 11.06 min) to give the title compound **1** (15 mg, yield: 25%).

MS(ESI) m/z = 374.2 [M+H]⁺.

¹H-NMR (400 MHz, DMSO-*d₆*) δ 8.87 (s, 1H), 8.78 (s, 1H),7.44-7.37 (m, 1H), 4.02 (s, 3H).

¹⁹F-NMR (376 MHz, DMSO-*d₆*) δ -132.28(dd, 1F), -142.60 (dd, 1F), -151.65 (dd, 1F).

### Example 2

### 6-Chloro-3-(6-chloro-2,4-difluoro-3-methoxyphenyl)thieno[3,2-b]pyridine-2-carboxylic acid (2); 6-Chloro-3-(6-chloro-2,4-difluoro-3-methoxyphenyl)thieno[3,2-b]pyridine-2-carboxylic acid (2-P1); 6-Chloro-3-(6-chloro-2,4-difluoro-3-methoxyphenyl)thieno[3,2-b]pyridine-2-carboxylic acid (2-P2)

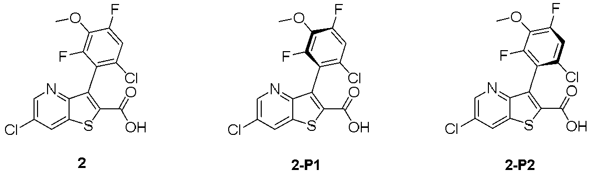

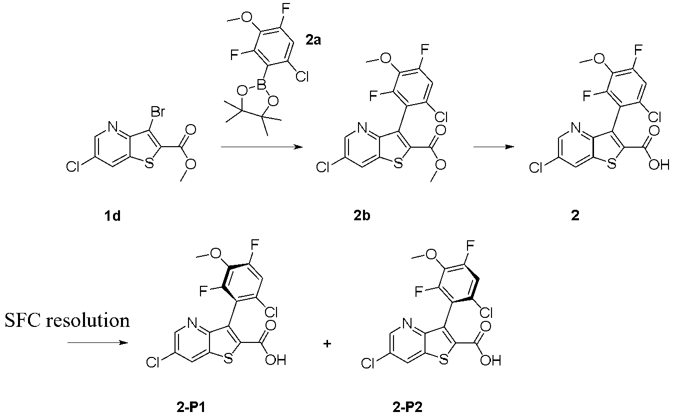

### Step 1: synthesis of methyl 6-chloro-3-(6-chloro-2,4-difluoro-3-methoxyphenyl)thieno[3,2-b]pyridine-2-carboxylate 2b

At room temperature, compound **1d** (60 mg, 0.20 mmol) and compound **2a** (89 mg, 0.30 mmol) were dissolved in toluene (3 mL) and water (0.3 mL), and tris(dibenzylideneacetone)dipalladium(0) (18 mg, 0.02 mmol), 2-dicyclohexylphosphino-2',6'-dimethoxybiphenyl (16 mg, 0.04 mmol), and potassium phosphate (125 mg, 0.59 mmol) were added. The reaction mixture was stirred at 70 °C for 2 h in a nitrogen atmosphere. After the mixture was cooled to room temperature, ethyl acetate (40 mL) was added. The mixture was washed with saturated brine (2 × 20 mL), dried over anhydrous sodium sulfate, filtered, and concentrated under reduced pressure to give a crude product, which was purified by column chromatography (petroleum ether/ethyl acetate) to give the title compound **2b** (50 mg, yield: 61%).

MS(ESI) m/z = 404.2 [M+H]⁺.

### Step 2: synthesis of 6-chloro-3-(6-chloro-2,4-difluoro-3-methoxyphenyl)thieno[3,2-b]pyridine-2-carboxylic acid 2

At room temperature, compound **2b** (50 mg, 0.12 mmol) was dissolved in a mixed solvent of tetrahydrofuran (2 mL), methanol (1 mL), and water (1 mL), and lithium hydroxide (30 mg, 1.2 mmol) was added. The reaction mixture was stirred at room temperature for 1 h. Water (30 mL) was added. The pH was adjusted to 4-5 with hydrochloric acid (1 N), followed by extraction with ethyl acetate (2 × 10 mL). The organic phases were combined, washed with saturated brine (2 × 10 mL), dried over anhydrous sodium sulfate, filtered, and concentrated under reduced pressure to give a crude product, which was separated and purified by preparative high performance liquid chromatography (column: Waters Xbridge, 30 × 250 mm; mobile phase A: 0.1% formic acid in water; mobile phase B: acetonitrile; gradient: 40%-95%, 18 min; flow rate: 30 mL/min; peak time: 11.06 min) to give the title compound **2** (18 mg, yield: 38%).

MS(ESI) m/z = 390.1 [M+H]⁺.

¹H-NMR (400 MHz, DMSO-*d₆*) δ 8.88 (s, 1H), 8.76 (s, 1H),7.63-7.60 (m, 1H), 3.96 (s, 3H).

¹⁹F-NMR (376 MHz, DMSO-*d₆*) δ -124.92 (d, 1F), -126.84(d, 1F).

### Step 3: 6-chloro-3-(6-chloro-2,4-difluoro-3-methoxyphenyl)thieno[3,2-b]pyridine-2-carboxylic acid (2-P1) and 6-chloro-3-(6-chloro-2,4-difluoro-3-methoxyphenyl)thieno[3,2-b]pyridine-2-carboxylic acid (2-P2)

The purified product **2** of step 2 was further resolved by SFC. Resolution method: column: ChiralPak AD, 250 × 30 mm I.D., 5 µm; mobile phase: A: CO₂; B: isopropanol [0.1% NH₃ (7 M in MeOH)]; gradient: B 30%; flow rate: 100 mL/min; back pressure: 100 bar; column temperature: 35 °C; wavelength: 214 nm; cycle time: about 2 min. Analysis method: column: ChiralPak AD, 100 × 4.6 mm I.D., 3 µm; mobile phase: A: CO₂; B: isopropanol (0.1% DEA); gradient: B 5%-45%, 4 min; flow rate: 3.0 mL/min; back pressure: 2000 psi; column temperature: 40 °C; wavelength: 214 nm; cycle time: about 2 min.
**2-P1,** retention time t = 2.985 min, 1.7 mg;
**2-P2,** retention time t = 2.807 min, 2.1 mg.

### Example 3

### 6-Chloro-3-(2,4,5-trifluoro-3-methylphenyl)thieno[3,2-b]pyridine-2-carboxylic acid (3)

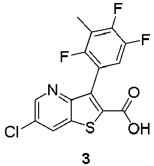

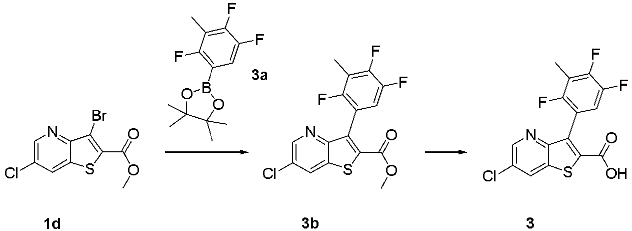

### Step 1: synthesis of methyl 6-chloro-3-(2,4,5-trifluoro-3-methylphenyl)thieno[3,2-b]pyridine-2-carboxylate 3b

At room temperature, compound **1d** (100 mg, 0.33 mmol) and compound **3a** (133 mg, 0.49 mmol) were dissolved in 1,4-dioxane (2 mL) and water (0.40 mL), and 1,1'-di-tert-butylphosphinoferrocene palladium(II) dichloride (21.3 mg, 0.03 mmol) and potassium carbonate (135 mg, 0.09 mmol) were added. The reaction mixture was stirred at 70 °C for 1 h in a nitrogen atmosphere. After the mixture was cooled to room temperature, ethyl acetate (40 mL) was added. The mixture was washed with saturated brine (2 × 20 mL), dried over anhydrous sodium sulfate, filtered, and concentrated under reduced pressure to give a crude product, which was purified by column chromatography (petroleum ether/ethyl acetate) to give the title compound **3b** (80 mg, yield: 65%).

MS(ESI) m/z = 372.2 [M+H]⁺.

### Step 2: synthesis of 6-chloro-3-(2,4,5-trifluoro-3-methylphenyl)thieno[3,2-b]pyridine-2-carboxylic acid 3

At room temperature, compound **3b** (80 mg, 0.22 mmol) was dissolved in a mixed solvent of tetrahydrofuran (2 mL), methanol (1 mL), and water (1 mL), and lithium hydroxide (52 mg, 2.2 mmol) was added. The reaction mixture was stirred at room temperature for 1 h. Water (30 mL) was added. The pH was adjusted to 4-5 with hydrochloric acid (1 N), followed by extraction with ethyl acetate (2 × 10 mL). The organic phases were combined, washed with saturated brine (2 × 20 mL), dried over anhydrous sodium sulfate, filtered, and concentrated under reduced pressure to give a crude product, which was separated and purified by preparative high performance liquid chromatography (column: Waters Xbridge, 30 × 250 mm; mobile phase A: 0.1% formic acid in water; mobile phase B: acetonitrile; gradient: 35%-85%, 18 min; flow rate: 30 mL/min; peak time: 13.50 min) to give the title compound **3** (18 mg, yield: 22%).

MS(ESI) m/z = 358.2 [M+H]⁺.

¹H-NMR (400 MHz, DMSO-*d₆*) δ 8.86 (s, 1H), 8.77 (s, 1H), 7.54-7.48 (m, 1H),2.26 (s, 3H).

¹⁹F-NMR (376 MHz, DMSO-*d₆*) δ -118.78(dd, 1F), -137.93(dd, 1F), -144.56 (dd, 1F).

### Example 4

### 6-Fluoro-3-[2,4,5-trifluoro-3-(methylthio)phenyl]thieno[3,2-b]pyridine-2-carboxylic acid (4)

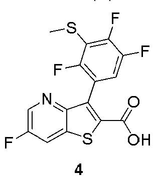

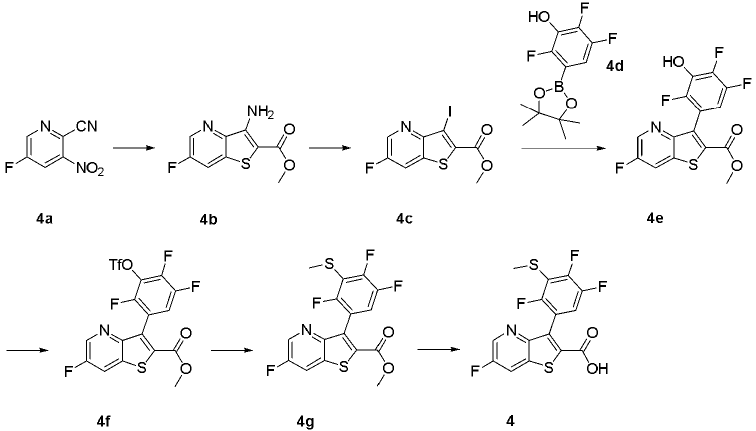

### Step 1: synthesis of methyl 3-amino-6-fluorothieno[3,2-b]pyridine-2-carboxylate 4b

At 0 °C, methyl mercaptoacetate (1.1 mL, 12.57 mmol) was added dropwise to a solution of compound **4a** (2 g, 11.97 mmol) in N,N-dimethylformamide (20 mL), and then an aqueous solution of potassium hydroxide (5 M, 4.8 mL, 23.94 mmol) was slowly added dropwise. The mixture was left to react at 0 °C for 30 min. The reaction mixture was poured into ice water (60 mL) and extracted with ethyl acetate (3 × 50 mL). The organic phases were combined, washed with saturated brine (50 mL), dried over anhydrous sodium sulfate, filtered, and concentrated under reduced pressure, and the resulting crude product was purified by column chromatography (petroleum ether/ethyl acetate) to give compound **4b** (2 g, yield: 73%).

MS(ESI) m/z = 227.2 [M+H]⁺.

### Step 2: synthesis of methyl 6-fluoro-3-iodothieno[3,2-b]pyridine-2-carboxylate 4c

At room temperature, compound **4b** (1 g, 4.42 mmol) and diiodomethane (0.54 mL, 6.63 mmol) were dissolved in acetonitrile (20 mL). The mixture was heated to 70 °C, and then isoamyl nitrite (0.89 mL, 6.63 mmol) was slowly added dropwise to the system, with the temperature maintained at 70-80 °C. After the addition, the mixture was left to react at 70 °C for 2 h. The reaction mixture was directly concentrated under reduced pressure to dryness, and the residue was purified by column chromatography (petroleum ether/ethyl acetate) to give a crude product of **4c** (350 mg, yield: 23%).

MS(ESI) m/z = 338.2 [M+H]⁺.

### Step 3: synthesis of methyl 6-fluoro-3-(2,4,5-trifluoro-3-hydroxyphenyl)thieno[3,2-b]pyridine-2-carboxylate 4e

At room temperature, compound **4c** (300 mg, 0.89 mmol) and compound **4d** (366 mg, 1.34 mmol) were dissolved in 1,4-dioxane (2 mL) and water (0.40 mL), and 1,1'-di-tert-butylphosphinoferrocene palladium(II) dichloride (58 mg, 0.09 mmol) and potassium carbonate (369 mg, 2.67 mmol) were added. The reaction mixture was stirred at 100 °C for 1 h in a nitrogen atmosphere. After the mixture was cooled to room temperature, ethyl acetate (20 mL) was added. The mixture was washed with saturated brine (2 × 10 mL), dried over anhydrous sodium sulfate, filtered, and concentrated under reduced pressure to give a crude product, which was purified by column chromatography (petroleum ether/ethyl acetate) to give the title compound **4e** (100 mg, yield: 31%).

MS(ESI) m/z = 358.2 [M+H]⁺.

### Step 4: synthesis of 2,5,6-trifluoro-3-[6-fluoro-2-(methoxycarbonyl)thieno[3,2-b]pyridin-3-yl]phenyl trifluoromethanesulfonate 4f

At room temperature, triethylamine (85 mg, 0.84 mmol) and trifluoromethanesulfonic anhydride (118 mg, 0.42 mmol) were added to a solution of compound **4e** (100 mg, 0.28 mmol) in dichloromethane (4 mL). After the addition, the mixture was left to react at room temperature for 1 h. The reaction mixture was concentrated under reduced pressure to remove dichloromethane, and ethyl acetate (20 mL) was added to the residue. The mixture was washed with saturated brine (2 × 10 mL), dried over anhydrous sodium sulfate, filtered, and concentrated under reduced pressure to give a crude product, which was purified by column chromatography (petroleum ether/ethyl acetate) to give the title compound **4f** (98 mg, yield: 71%).

MS(ESI) m/z = 490.2 [M+H]⁺.

### Step 5: synthesis of methyl 6-fluoro-3-[2,4,5-trifluoro-3-(methylthio)phenyl]thieno[3,2-b]pyridine-2-carboxylate 4g

At room temperature, compound **4f** (80 mg, 0.16 mmol), sodium thiomethoxide (57 mg, 0.82 mmol), palladium acetate (7.3 mg, 0.033 mmol), 2,2'-bis(diphenylphosphino)-1,1'-binaphthyl (20.4 mg, 0.033 mmol), and toluene (2 mL) were added to a round-bottom flask. In a nitrogen atmosphere, the mixture was heated to 120 °C and left to react overnight. After the reaction mixture was cooled to room temperature, ethyl acetate (20 mL) was added. The mixture was washed with saturated brine (2 × 10 mL), dried over anhydrous sodium sulfate, filtered, and concentrated under reduced pressure to give a crude product, which was purified by column chromatography (petroleum ether/ethyl acetate) to give the title compound **4g** (50 mg, yield: 79%).

MS(ESI) m/z = 388.2 [M+H]⁺.

### Step 6: synthesis of 6-fluoro-3-[2,4,5-trifluoro-3-(methylthio)phenyl]thieno[3,2-b]pyridine-2-carboxylic acid 4

At room temperature, compound **4g** (50 mg, 0.13 mmol) was dissolved in a mixed solvent of tetrahydrofuran (2 mL), methanol (1 mL), and water (1 mL), and lithium hydroxide (31 mg, 1.3 mmol) was added. The reaction mixture was stirred at room temperature for 1 h. Water (30 mL) was added. The pH was adjusted to 4-5 with hydrochloric acid (1 N, 4 mL), followed by extraction with ethyl acetate (2 × 20 mL). The organic phases were combined, washed with saturated brine (2 × 20 mL), dried over anhydrous sodium sulfate, filtered, and concentrated under reduced pressure to give a crude product, which was separated and purified by preparative high performance liquid chromatography (column: Waters Xbridge, 30 × 250 mm; mobile phase A: 0.1% formic acid in water; mobile phase B: acetonitrile; gradient: 40%-95%, 18 min; flow rate: 30 mL/min; peak time: 10.99 min) to give the title compound **4** (18 mg, yield: 37%).

MS(ESI) m/z = 374.2 [M+H]⁺.

¹H-NMR (400 MHz, DMSO-*d₆*) δ 8.80 (s, 1H), 8.65 (d, 1H), 2.52 (s, 3H).

¹⁹F-NMR (376 MHz, DMSO-*d₆*) δ -110.02 (d, 1F), -127.38(s, 1F), -129.29 (d, 1F), - 142.40 (dd, 1F).

### Example 5

### 6-Fluoro-3-(2,4,5-trifluoro-3-methoxyphenyl)thieno[3,2-b]pyridine-2-carboxylic acid (5)

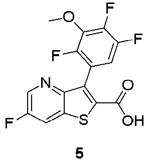

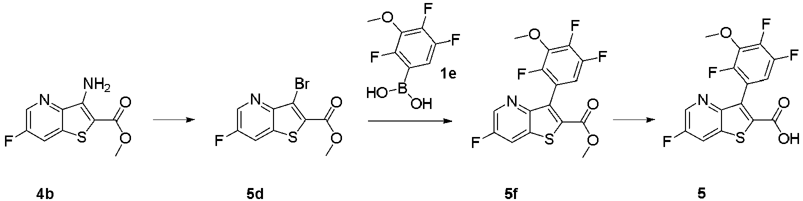

6-Fluoro-3-(2,4,5-trifluoro-3-methoxyphenyl)thieno[3,2-b]pyridine-2-carboxylic acid (**5**) was prepared by using the synthesis method in Example 1 and replacing compound **1d** with compound **4b.**

Preparative high performance liquid chromatography separation and purification conditions for compound **5:** column: Waters Xbridge, 30 × 250 mm; mobile phase A: 0.1% formic acid in water; mobile phase B: acetonitrile; gradient: 40%-90%, 18 min; flow rate: 30 mL/min; peak time: 10.5 min.

MS(ESI) m/z = 358.30 [M+H]⁺.

¹H-NMR(400 MHz, DMSO-*d*₆) δ 8.80 (d, 1H), 8.64 (dd, 1H), 7.44-7.38 (m, 1H), 4.01(s, 3H).

¹⁹F-NMR(376 MHz, DMSO-*d*₆) δ -127.42 (s, 1F), -132.32 (dd, 1F), -142.65 (dd, 1F), - 151.72 (dd, 1F).

### Example 6

### 3-(3-Ethyl-2,4,5-trifluorophenyl)-6-fluorothieno[3,2-b]pyridine-2-carboxylic acid (6)

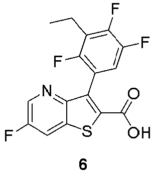

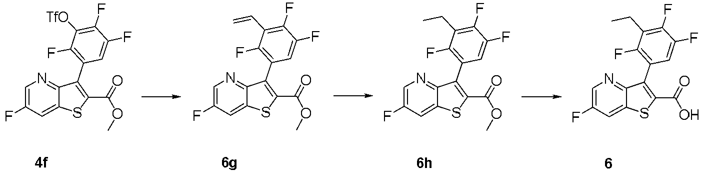

### Step 1: synthesis of methyl 6-fluoro-3-(2,4,5-trifluoro-3-vinylphenyl)thieno[3,2-b]pyridine-2-carboxylate 6g

At room temperature, compound **4f** (160 mg, 0.32 mmol), potassium vinyltrifluoroborate (127 mg, 0.95 mmol), tris(dibenzylideneacetone)dipalladium(0) (28.9 mg, 0.032 mmol), 2-dicyclohexylphosphino-2',6'-dimethoxybiphenyl (13 mg, 0.032 mmol), and potassium phosphate (168 mg, 0.79 mmol) were dissolved in toluene (5 mL). The mixture was left to react at 100 °C for 1 h in a nitrogen atmosphere. The reaction mixture was poured into water (50 mL) and extracted with ethyl acetate (3 × 30 mL). The organic phases were combined, washed with saturated brine (30 mL), dried over anhydrous sodium sulfate, filtered, and concentrated under reduced pressure. The residue was purified by column chromatography (petroleum ether/ethyl acetate) to give **6g** (112 mg, yield: 95%).

MS(ESI) m/z = 368.2 [M+H]⁺.

### Step 2: synthesis of methyl 3-(3-ethyl-2,4,5-trifluorophenyl)-6-fluorothieno[3,2-b]pyridine-2-carboxylate 6h

At room temperature, compound **6g** (100 mg, 0.26 mmol) and 10% palladium on carbon (29 mg, 0.27 mmol) were dissolved in methanol (15 mL). The mixture was stirred for 2 h in a hydrogen atmosphere. The reaction mixture was directly filtered, and the filtrate was concentrated under reduced pressure to give a crude product of **6h** (95 mg, 98%).

MS(ESI) m/z = 370.2 [M+H]⁺.

### Step 3: synthesis of 3-(3-ethyl-2,4,5-trifluorophenyl)-6-fluorothieno[3,2-b]pyridine-2-carboxylic acid 6

At room temperature, compound **6h** (100 mg, 0.27 mmol) was dissolved in a mixed solvent of tetrahydrofuran (6 mL), methanol (3 mL), and water (3 mL), and lithium hydroxide (114 mg, 2.71 mmol) was added. The reaction mixture was stirred at room temperature for 1 h. Water (30 mL) was added. The pH was adjusted to 4-5 with hydrochloric acid (1 N, 4 mL), followed by extraction with ethyl acetate (3 × 30 mL). The organic phases were combined, washed with saturated brine (2 × 20 mL), dried over anhydrous sodium sulfate, filtered, and concentrated under reduced pressure to give a crude product, which was separated and purified by preparative high performance liquid chromatography (column: Waters Xbridge, 30 × 250 mm; mobile phase A: 0.1% formic acid in water; mobile phase B: acetonitrile; gradient: 40%-95%, 18 min; flow rate: 30 mL/min; peak time: 11.52 min) to give the title compound **6** (26.8 mg, yield: 27%).

MS(ESI) m/z = 356.2 [M+H]⁺.

¹H-NMR(400 MHz, DMSO-*d*₆) δ 8.79 (s, 1H), 8.63 (d, 1H), 7.52 (dd, 1H), 2.73 (q, 2H), 1.19(q, 3H).

¹⁹F-NMR(376 MHz, DMSO-*d*₆) δ -120.81 (dd, 1F), -127.60 (s, 1F), -140.39 (dd, 1F) - 144.30 (dd, 1F).

### Example 7

### 6-Chloro-3-(3-ethyl-2,4,5-trifluorophenyl)thieno[3,2-b]pyridine-2-carboxylic acid (7)

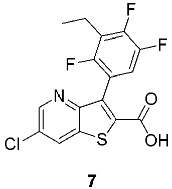

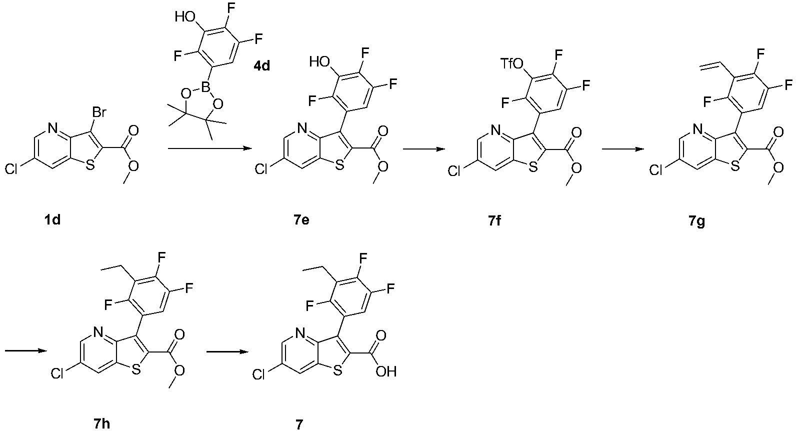

### Step 1: synthesis of methyl 6-chloro-3-(2,4,5-trifluoro-3-hydroxyphenyl)thieno[3,2-b]pyridine-2-carboxylate 7e

At room temperature, compound **1d** (500 mg, 1.64 mmol) and compound **4d** (676 mg, 2.47 mmol) were dissolved in 1,4-dioxane (5 mL) and water (0.80 mL), and 1,1'-di-tert-butylphosphinoferrocene palladium(II) dichloride (103 mg, 0.16 mmol) and potassium carbonate (680 mg, 4.92 mmol) were added. The reaction mixture was stirred at 100 °C for 1 h in a nitrogen atmosphere. After the mixture was cooled to room temperature, ethyl acetate (40 mL) was added. The mixture was washed with saturated brine (2 × 15 mL), dried over anhydrous sodium sulfate, filtered, and concentrated under reduced pressure to give a crude product, which was purified by column chromatography (petroleum ether/ethyl acetate) to give the title compound **7e** (171 mg, yield: 28%).

MS(ESI) m/z = 374.1 [M+H]⁺.

### Step 2: synthesis of 2,5,6-trifluoro-3-[6-chloro-2-(methoxycarbonyl)thieno[3,2-b]pyridin-3-yl]phenyl trifluoromethanesulfonate 7f

At room temperature, triethylamine (255 mg, 1.38 mmol) and trifluoromethanesulfonic anhydride (177 mg, 0.69 mmol) were added to a solution of compound **7e** (171 mg, 0.46 mmol) in dichloromethane (6 mL). After the addition, the mixture was left to react at room temperature for 1 h. The reaction mixture was concentrated under reduced pressure to remove dichloromethane, and ethyl acetate (40.0 mL) was added to the residue. The mixture was washed with saturated brine (2 × 20 mL), dried over anhydrous sodium sulfate, filtered, and concentrated under reduced pressure to give a crude product, which was purified by column chromatography (petroleum ether/ethyl acetate) to give the title compound **7f** (200 mg, yield: 86%).

MS(ESI) m/z = 506.1 [M+H]⁺.

6-Chloro-3-(3-ethyl-2,4,5-trifluorophenyl)thieno[3,2-b]pyridine-2-carboxylic acid (7) was synthesized from compound **7f** in three steps by using the synthesis method in Example 6.

Preparative high performance liquid chromatography separation and purification conditions for compound 7: column: Waters Xbridge, 30 × 250 mm; mobile phase A: 0.1% formic acid in water; mobile phase B: acetonitrile; gradient: 45%-95%, 18 min; flow rate: 30 mL/min; peak time: 12.37 min.

MS(ESI) m/z = 372.1 [M+H]⁺.

¹H-NMR(400 MHz, DMSO-*d*₆) δ 8.86 (s, 1H), 8.78 (s, 1H), 7.52 (q, 1H), 2.73 (q, 2H), 1.19 (t, 3H).

¹⁹F-NMR(376 MHz, DMSO-*d*₆) δ -120.80 (dd, 1F), -140.28 (dd, 1F), -144.24 (dd, 1F).

### Example 8

### 3-{6-chloro-3-[(difluoromethyl)oxy]-2,4-difluorophenyl}-6-fluorothieno[3,2-b]pyridine-2-carboxylic acid (8)

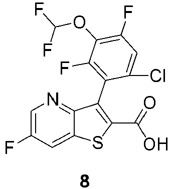

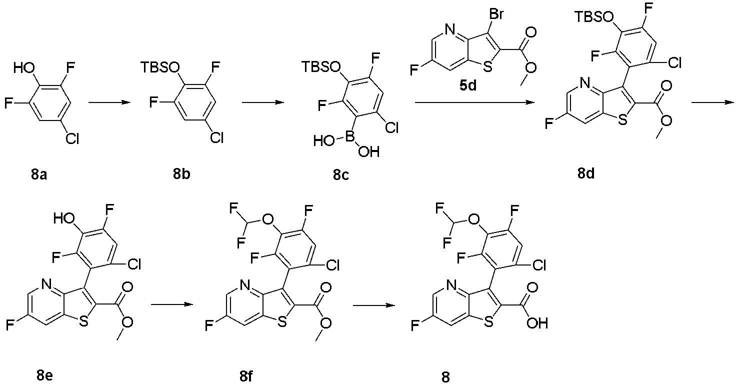

### Step 1: synthesis of tert-butyl (4-chloro-2,6-difluorophenoxy)dimethylsilane 8b

Triethylamine (9.2 g, 91.2 mmol) was added to a solution of compound **8a** (10 g, 60.8 mmol) in dichloromethane (200 mL) in an ice bath. After 10 min of stirring, tertbutyldimethylsilyl trifluoromethanesulfonate (24.1 g, 91.2 mmol) was added dropwise. After the addition, the mixture was stirred at room temperature for 1 h. The reaction mixture was washed with saturated brine (3 × 80 mL), dried over anhydrous sodium sulfate, filtered, and concentrated under reduced pressure. The residue was purified by column chromatography (petroleum ether/ethyl acetate) to give **8b** (15.1 g, yield: 89%) in the form of an oil.

### Step 2: synthesis of (3-((tert-butyldimethylsilyl)oxy)-6-chloro-2,4-difluorophenyl)boronic acid 8c

At -78 °C, in a nitrogen atmosphere, n-butyllithium (2.5 M, 23.6 mL, 59.1 mmol) was slowly added dropwise to a solution of compound **8b** (15 g, 53.8 mmol) in anhydrous tetrahydrofuran (200 mL). After the addition, the mixture was stirred at -78 °C for 1 h, and then a solution of trimethyl borate (7.3 g, 70 mmol) in anhydrous tetrahydrofuran (20 mL) was slowly added dropwise. After the addition, the mixture was stirred at -78 °C for another 1 h. The reaction mixture was slowly added to a saturated ammonium chloride solution (200 mL) stirred in an ice bath, and after 5 min, ethyl acetate (100 mL) was added. After the mixture was mixed well, the organic phase was isolated, washed with saturated brine (3 × 80 mL), dried over anhydrous sodium sulfate, filtered, and concentrated under reduced pressure. The residue was purified by column chromatography (petroleum ether/ethyl acetate) to give **8c** as an oil (11.2 g, yield: 64%).

¹H-NMR (400 MHz, DMSO-*d₆*) δ 8.71 (s, 2H), 7.28 (d, 1H), 0.98 (s, 9H), 0.18 (s, 6H).

### Step 3: synthesis of methyl 3-(3-((tert-butyldimethylsilyl)oxy)-6-chloro-2,4-difluorophenyl)-6-fluorothieno[3,2-b]pyridine-2-carboxylate 8d

At room temperature, compound **8c** (334 mg, 1.03 mmol) and compound **5d** (300 mg, 1.03 mmol) were dissolved in toluene (5 mL) and water (1 mL), and tris(dibenzylideneacetone)dipalladium(0) (94.6 mg, 0.10 mmol), 2-dicyclohexylphosphino-2',6'-dimethoxybiphenyl (84.9 mg, 0.20 mmol), and potassium phosphate (548 mg, 2.6 mmol) were added. The reaction mixture was stirred at 100 °C for 1 h in a nitrogen atmosphere. After the mixture was cooled to room temperature, ethyl acetate (40 mL) was added. The mixture was washed with saturated brine (2 × 20 mL), dried over anhydrous sodium sulfate, filtered, and concentrated under reduced pressure to give a crude product, which was purified by column chromatography (petroleum ether/ethyl acetate) to give the title compound **8d** (197 mg, yield: 39%).

MS(ESI) m/z = 488.4 [M+H]⁺.

### Step 4: synthesis of methyl 3-(6-chloro-2,4-difluoro-3-hydroxyphenyl)-6-fluorothieno[3,2-b]pyridine-2-carboxylate 8e

At room temperature, compound **8d** (180 mg, 0.37 mmol) and tetrabutylammonium fluoride (1 M, 0.6 mL, 0.6 mmol) were dissolved in tetrahydrofuran (10 mL). The mixture was stirred at room temperature for 2 h. The reaction mixture was poured into water (30 mL) and extracted with ethyl acetate (3 × 20 mL). The organic phases were combined, washed with saturated brine (20 mL), dried over anhydrous sodium sulfate, filtered, and concentrated under reduced pressure to give a crude product of **8e** (201 mg, crude product yield: 145%).

MS(ESI) m/z = 374.2 [M+H]⁺.

### Step 5: synthesis of methyl 3-{6-chloro-3-[(difluoromethyl)oxy]-2,4-difluorophenyl}-6-fluorothieno[3,2-b]pyridine-2-carboxylate 8f

At room temperature, compound **8e** (180 mg, 0.48 mmol), sodium difluorochloroacetate (147 mg, 0.96 mmol), and potassium carbonate (80 mg, 0.58 mmol) were dissolved in N,N-dimethylformamide (5 mL). The mixture was left to react at 100 °C for 2 h in a nitrogen atmosphere. The reaction mixture was poured into water (30 mL) and extracted with ethyl acetate (3 × 20 mL). The organic phases were combined, washed with saturated brine (20 mL), dried over anhydrous sodium sulfate, filtered, and concentrated under reduced pressure to give a crude product, which was then purified by column chromatography (petroleum ether/ethyl acetate) to give compound **8f** (100 mg, 49%).

MS(ESI) m/z = 424.2 [M+H]⁺.

### Step 6: synthesis of 3-{6-chloro-3-[(difluoromethyl)oxy]-2,4-difluorophenyl}-6-fluorothieno[3,2-b]pyridine-2-carboxylic acid 8

At room temperature, compound **8f** (100 mg, 0.236 mmol) was dissolved in a mixed solvent of tetrahydrofuran (4 mL), methanol (2 mL), and water (2 mL), and lithium hydroxide (100 mg, 2.4 mmol) was added. The reaction mixture was stirred at room temperature for 2 h. Water (30 mL) was added. The pH was adjusted to 4-5 with hydrochloric acid (1 N), followed by extraction with ethyl acetate (2 × 30 mL). The organic phases were combined, washed with saturated brine (2 × 20 mL), dried over anhydrous sodium sulfate, filtered, and concentrated under reduced pressure to give a crude product, which was separated and purified by preparative high performance liquid chromatography (column: Waters Xbridge, 30 × 250 mm; mobile phase A: 0.1% formic acid in water; mobile phase B: acetonitrile; gradient: 35%-90%, 18 min; flow rate: 30 mL/min; peak time: 11.37 min) to give the title compound **8** (27.8 mg, yield: 28%).

MS(ESI) m/z = 410.1 [M+H]⁺.

¹H-NMR(400 MHz, DMSO-*d*₆) δ 8.78 (s, 1H), 8.67 (d, 1H), 7.84 (d, 1H), 7.34 (t, 1H).

¹⁹F-NMR(376 MHz, DMSO-*d*₆) δ -82.76 (t, 2F), -121.98 (dd, 1F), -124.08 (s, 1F), - 126.95 (s, 1F).

### Example 9

### 3-{3-[(Difluoromethyl)oxy]-2,4,5-trifluorophenyl}-6-fluorothieno[3,2-b]pyridine-2-carboxylic acid (9)

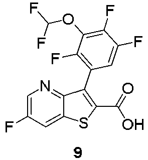

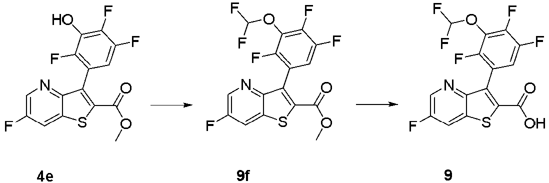

3-{3-[(Difluoromethyl)oxy]-2,4,5-trifluorophenyl}-6-fluorothieno[3,2-b]pyridine-2-carboxylic acid (**9**) was prepared by using the synthesis method in Example 8 and replacing compound **8e** with compound **4e.**

Preparative high performance liquid chromatography separation and purification conditions for compound **9:** column: Waters Xbridge, 30 × 250 mm; mobile phase A: 0.1% formic acid in water; mobile phase B: acetonitrile; gradient: 25%-70%, 18 min; flow rate: 30 mL/min; peak time: 11.72 min.

MS(ESI) m/z = 392.1 [M-H]⁻.

¹H-NMR(400 MHz, DMSO-*d*₆) δ 8.81 (s, 1H), 8.65 (d, 1H), 7.76 (dd, 1H), 7.36 (t, 1H).

¹⁹F-NMR(376 MHz, DMSO-*d*₆) δ -82.48 (s, 2F), -127.30 (3, 1F), -129.43~-129.50 (m, 1F), -141.61 (dd, 1F), -147.72 (d, 1F).

### Example 10

### 6-Chloro-3-{3-[(difluoromethyl)oxy]-2,4,5-trifluorophenyl}thieno[3,2-b]pyridine-2-carboxylic acid (10)

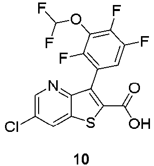

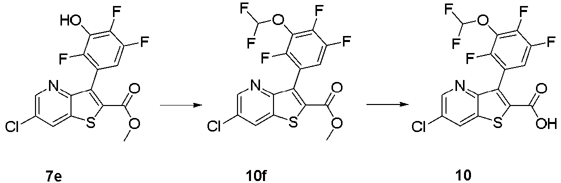

6-Chloro-3-{3-[(difluoromethyl)oxy]-2,4,5-trifluorophenyl}thieno[3,2-b]pyridine-2-carboxylic acid (**10**) was prepared by using the synthesis method in Example 8 and replacing compound **8e** with compound **7e.**

Preparative high performance liquid chromatography separation and purification conditions for compound **10:** column: Waters Xbridge, 30 × 250 mm; mobile phase A: 0.1% formic acid in water; mobile phase B: acetonitrile; gradient: 45%-90%, 18 min; flow rate: 30 mL/min; peak time: 11.28 min.

MS(ESI) m/z = 392.1 [M-H]⁻.

¹H-NMR(400 MHz, DMSO-*d*₆) δ 8.81 (s, 1H), 8.65 (d, 1H), 7.76 (dd, 1H), 7.36 (t, 1H).

¹⁹F-NMR(376 MHz, DMSO-*d*₆) δ -82.48 (s, 2F), -127.30 (3, 1F), -129.43~-129.50 (m, 1F), -141.61 (dd, 1F), -147.72 (d, 1F).

### Example 11

### 6-Chloro-3-{6-chloro-3-[(difluoromethyl)oxy]-2,4-difluorophenyl}thieno[3,2-b]pyridine-2-carboxylic acid (11)

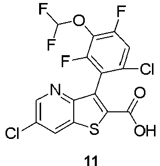

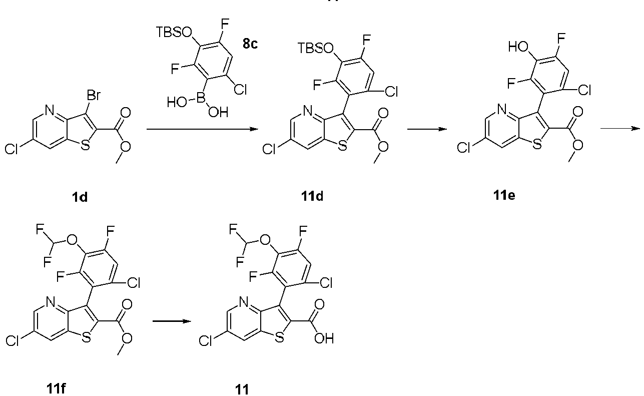

6-Chloro-3-{6-chloro-3-[(difluoromethyl)oxy]-2,4-difluorophenyl}thieno[3,2-b]pyridine-2-carboxylic acid (**11**) was prepared by using the synthesis method in Example 8 and replacing compound **5d** with compound **1d**.

Preparative high performance liquid chromatography separation and purification conditions for compound **11:** column: Waters Xbridge, 30 × 250 mm; mobile phase A: 0.1% formic acid in water; mobile phase B: acetonitrile; gradient: 50%-95%, 18 min; flow rate: 30 mL/min; peak time: 10.18 min.

MS(ESI) m/z = 426.1 [M+H]⁺.

¹H-NMR(400 MHz, DMSO-*d*₆) δ 8.89 (s, 1H), 8.77 (s, 1H), 7.83(d, 1H), 7.33(t, 1H).

¹⁹F-NMR(376 MHz, DMSO-*d*₆) δ -82.75(t, 2F), -121.93(d, 1F), -124.02(s, 1F).

### Example 12

### 6-Chloro-3-(4-chloro-2-fluoro-3-methoxyphenyl)thieno[3,2-b]pyridine-2-carboxylic acid (12)

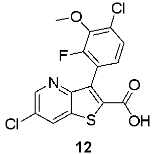

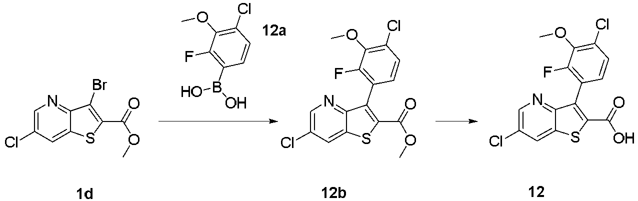

6-Chloro-3-(4-chloro-2-fluoro-3-methoxyphenyl)thieno[3,2-b]pyridine-2-carboxylic acid (**12**) was prepared by using the synthesis method in Example 8, replacing compound **5d** with compound **1d**, and replacing compound **8c** with compound **12a**.

Preparative high performance liquid chromatography separation and purification conditions for compound **12:** column: Waters Xbridge, 30 × 250 mm; mobile phase A: 0.1% formic acid in water; mobile phase B: acetonitrile; gradient: 50%-90%, 18 min; flow rate: 30 mL/min; peak time: 10.65 min.

MS(ESI) m/z = 372.2 [M+H]⁺.

¹H-NMR(400 MHz, DMSO-*d*₆) δ 8.86 (s, 1H), 8.76 (s, 1H), 7.43 (d, 1H), 7.24 (t, 1H), 3.90 (s, 3H).

¹⁹F-NMR(376 MHz, DMSO-*d*₆) δ -127.61 (s, 1F).

### Example 13

### 6-Chloro-3-(4-chloro-2-fluoro-3-methylphenyl)thieno[3,2-b]pyridine-2-carboxylic acid (13)

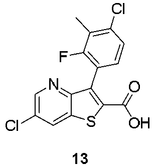

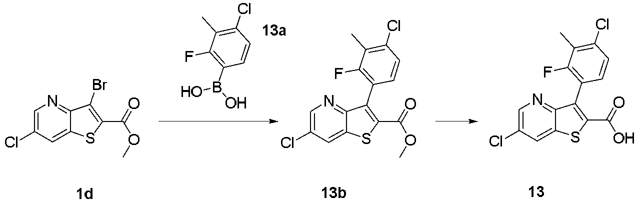

6-Chloro-3-(4-chloro-2-fluoro-3-methylphenyl)thieno[3,2-b]pyridine-2-carboxylic acid (**13**) was synthesized by using the synthesis method in Example 8.

Preparative high performance liquid chromatography separation and purification conditions for compound **13:** column: Waters Xbridge, 30 × 250 mm; mobile phase A: 0.1% formic acid in water; mobile phase B: acetonitrile; gradient: 50%-95%, 18 min; flow rate: 30 mL/min; peak time: 11.22 min.

MS(ESI) m/z = 356.1 [M+H]⁺.

¹H-NMR(400 MHz, DMSO-*d*₆) δ 8.85(s, 1H), 8.74(s, 1H), 7.40(d, 1H), 7.33(t, 1H), 2.31(s, 3H).

¹⁹F-NMR(376 MHz, DMSO-*d*₆) δ -112.58(s, 1F).

### Example 14

### 6-Chloro-3-{6-chloro-3-[(difluoromethyl)oxy]-2,4-difluorophenyl}thieno[3,2-b]pyridine-2-carboxylic acid (11-P1); 6-Chloro-3-{6-chloro-3-[(difluoromethyl)oxy]-2,4-difluorophenyl}thieno[3,2-b]pyridine-2-carboxylic acid (11-P2)

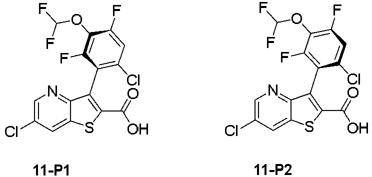

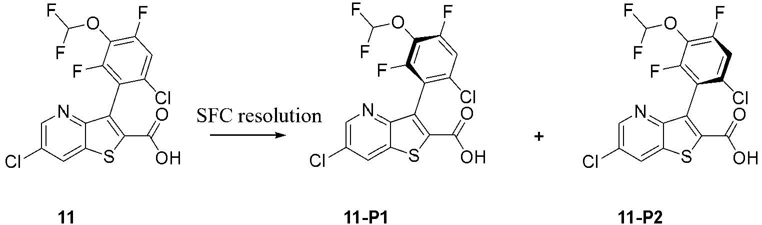

Compound **11** (60 mg) was resolved by SFC. Resolution method: column: ChiralPak IC, 250 × 30 mm I.D., 5 µm; mobile phase: A: CO₂; B: isopropanol [0.1% NH₃ (7 M in MeOH)]; gradient: B 20%; flow rate: 100 mL/min; back pressure: 100 bar; column temperature: 35 °C; wavelength: 214 nm; cycle time: about 2.5 min.
**11-P1,** retention time t = 2.862 min, 24 mg;
**11-P2,** retention time t = 2.263 min, 18 mg.

### Example 15

### 6-Chloro-3-(4-chloro-2,5-difluoro-3-methylphenyl)thieno[3,2-b]pyridine-2-carboxylic acid (15)

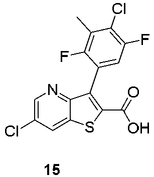

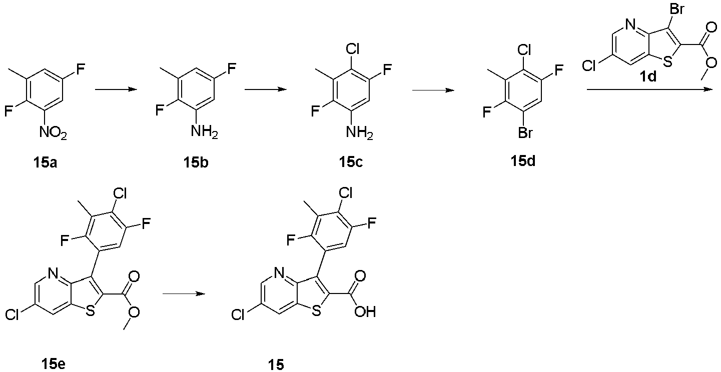

### Step 1: synthesis of 2,5-difluoro-3-methylaniline 15b

At room temperature, compound **15a** (1.00 g, 5.78 mmol), iron powder (0.97 g, 17.37 mmol), and ammonium chloride (1.54 g, 28.88 mmol) were added to ethanol (10 mL) and water (3 mL). The mixture was stirred at room temperature overnight in a nitrogen atmosphere. The reaction mixture was filtered. The filter residue was washed with ethyl acetate (3 × 30 mL), and the filtrate was concentrated under reduced pressure. The residue was purified by column chromatography (petroleum ether/ethyl acetate) to give compound **15b** (700 mg, yield: 84%).

MS(ESI) m/z = 144.1 [M+H]⁺.

### Step 2: synthesis of 4-chloro-2,5-difluoro-3-methylaniline 15c

At room temperature, compound **15b** (650 mg, 4.54 mmol) and N-chlorosuccinimide (606 mg, 4.54 mmol) were dissolved in dichloromethane (10 mL). The mixture was stirred at 50 °C overnight in a nitrogen atmosphere. The reaction mixture was concentrated under reduced pressure, and the residue was purified by column chromatography (petroleum ether/ethyl acetate) to give compound **15c** (300 mg, yield: 37%).

### Step 3: synthesis of 1-bromo-4-chloro-2,5-difluoro-3-methylbenzene 15d

At room temperature, tert-butyl nitrite (0.25 mL, 2.11 mmol) and copper bromide (408 mg, 1.83 mmol) were dissolved in acetonitrile (10 mL), and then compound **15c** (250 mg, 1.41 mmol) was added in portions to the above system. The mixture was stirred at room temperature for 1 h. The reaction mixture was poured into water (30 mL), extracted with ethyl acetate (3 × 30 mL), washed with saturated brine, dried over anhydrous sodium sulfate, filtered, and concentrated under reduced pressure. The residue was purified by column chromatography (petroleum ether/ethyl acetate) to give compound **15d** (300 mg, yield: 88%).

### Step 4: synthesis of methyl 6-chloro-3-(4-chloro-2,5-difluoro-3-methylphenyl)thieno[3,2-b]pyridine-2-carboxylate 15e

At room temperature, compound **15d** (200 mg, 0.83 mmol), bis(pinacolato)diboron (210 mg, 0.83 mmol), 1,1'-bis(diphenylphosphino)ferrocene palladium(II) dichloride (61.44 mg, 0.08 mmol), and potassium acetate (203 mg, 2.07 mmol) were dissolved in 1,4-dioxane (5 mL). The mixture was stirred at 80 °C overnight in a nitrogen atmosphere. Then, compound **1d** (100 mg, 0.33 mmol), potassium carbonate (239 mg, 1.73 mmol), 1,1'-di-tert-butylphosphinoferrocene palladium(II) dichloride (45 mg, 0.07 mmol), and water (1 mL) were added to the above system. The mixture was stirred at 80 °C for 1 h in a nitrogen atmosphere. The reaction mixture was poured into water (30 mL), extracted with ethyl acetate (3 × 30 mL), washed with saturated brine, dried over anhydrous sodium sulfate, and filtered, and the filtrate was concentrated under reduced pressure. The residue was purified by column chromatography (petroleum ether/ethyl acetate) to give compound **15e** (120 mg, yield: 44%).

### Step 5: synthesis of 6-chloro-3-(4-chloro-2,5-difluoro-3-methylphenyl)thieno[3,2-b]pyridine-2-carboxylic acid 15

At room temperature, compound **15e** (120 mg, 0.31 mmol) and lithium hydroxide (130 mg, 3.09 mmol) were dissolved in a mixed solvent of tetrahydrofuran (5 mL), methanol (2 mL), and water (2 mL). The mixture was stirred at room temperature for 1 h. Water (30 mL) was added. The pH was adjusted to 4-5 with hydrochloric acid (1 N, 4 mL), followed by extraction with ethyl acetate (2 × 20 mL). The organic phases were combined, washed with saturated brine (2 × 20 mL), dried over anhydrous sodium sulfate, and filtered. The filtrate was concentrated under reduced pressure to give a crude product, which was separated and purified by preparative high performance liquid chromatography (column: Waters Xbridge, 30 × 250 mm; mobile phase A: 0.1% formic acid in water; mobile phase B: acetonitrile; gradient: 45%-95%, 15 min; flow rate: 30 mL/min; peak time: 9.85 min) to give the title compound **15** (54 mg, yield: 46%).

MS(ESI) m/z = 374.1 [M+H]⁺.

¹H-NMR(400 MHz, DMSO-*d*₆) δ 8.86 (s, 1H), 8.76 (s, 1H), 7.50 (t, 1H), 2.35(s, 3H).

¹⁹F-NMR(376 MHz, DMSO-*d*₆) δ -117.41 (d, 1F), -120.46 (d, 1F).

### Example 16

### 6-Chloro-3-(5-chloro-2,4-difluoro-3-methoxyphenyl)thieno[3,2-b]pyridine-2-carboxylic acid (16)

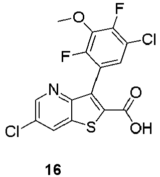

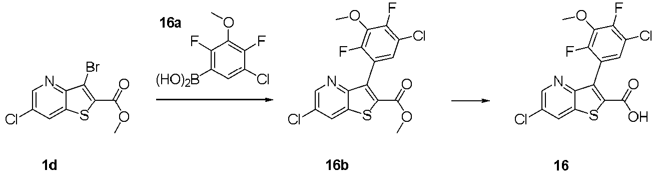

6-Chloro-3-(5-chloro-2,4-difluoro-3-methoxyphenyl)thieno[3,2-b]pyridine-2-carboxylic acid (**16**) was prepared by using the synthesis method in Example 1 and replacing compound **1e** with compound **16a.**

Preparative high performance liquid chromatography separation and purification conditions for compound **16:** column: Waters Xbridge, 30 × 250 mm; mobile phase A: 0.1% formic acid in water; mobile phase B: acetonitrile; gradient: 40%-95%, 18 min; flow rate: 30 mL/min; peak time: 12.26 min.

MS(ESI) m/z = 390.1 [M+H]⁺.

¹H-NMR(400 MHz, DMSO-*d*₆) δ 8.87 (s, 1H), 8.79 (s, 1H), 7.50 (t, 1H), 3.99 (s, 3H).

¹⁹F-NMR(376 MHz, DMSO-*d*₆) δ -127.96 (s, 1F), -128.99 (s, 1F).

### Example 17

### 6-Chloro-3-(5-chloro-2,4-difluoro-3-methylphenyl)thieno[3,2-b]pyridine-2-carboxylic acid (17)

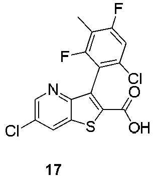

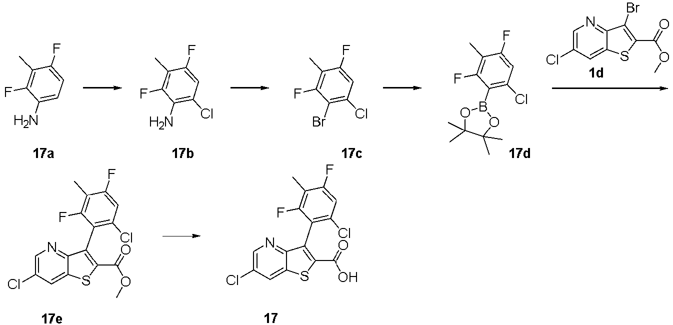

### Step 1: synthesis of 6-chloro-2,4-difluoro-3-methylaniline 17b

At room temperature, compound **17a** (2 g, 13.97 mmol) and N-chlorosuccinimide (1.96 mg, 14.67 mmol) were dissolved in dichloromethane (20 mL). The mixture was stirred at 50 °C overnight and concentrated to remove the solvent. Ethyl acetate (50 mL) was added to the residue. The mixture was then washed with water (1 × 10 mL) and saturated brine (2 × 10 mL), dried over anhydrous sodium sulfate, and concentrated under reduced pressure to give a crude product, which was then purified by column chromatography (petroleum ether/ethyl acetate) to give **17b** (700 mg, yield: 28%).

### Step 2: synthesis of 2-bromo-1-chloro-3,5-difluoro-4-methylbenzene 17c

At room temperature, copper bromide (973 mg, 4.34 mmol) and acetonitrile (15 mL) were added to a three-necked flask, and tert-butyl nitrite (528 mg, 5.12 mmol) was added dropwise in a nitrogen atmosphere. At 20 °C, a suspension of compound **17b** (700 mg, 3.94 mmol) in acetonitrile (4 mL) was added dropwise. After the addition, the mixture was stirred at room temperature for 2 h. Diluted hydrochloric acid (1 N, 10 mL) was added, followed by extraction with ethyl acetate (2 × 20 mL). The mixture was washed with water (1 × 10 mL) and saturated brine (2 × 10 mL) in sequence, dried over anhydrous sodium sulfate, filtered, and concentrated under reduced pressure to give a crude product, which was then purified by column chromatography (petroleum ether/ethyl acetate) to give compound **17c** (450 mg, yield: 47%).

### Step 3: synthesis of 2-(6-chloro-2,4-difluoro-3-methylphenyl)-4,4,5,5-tetramethyl-1,3,2-dioxaborolane 17d

In a nitrogen atmosphere, at the temperature of a dry ice-acetonitrile system, a solution of isopropylmagnesium chloride in tetrahydrofuran (2 M, 2.8 mL) was slowly added dropwise to a solution of compound **17c** (450 mg, 1.86 mmol) in tetrahydrofuran (5 mL). After 3 h of stirring, 4,4,5,5-tetramethyl-2-(propan-2-yloxy)-1,3,2-dioxaborolane (0.76 mL, 3.73 mmol) was slowly added dropwise to the reaction mixture. After the addition, the mixture was slowly heated to 20 °C and stirred for 2 h. Diluted hydrochloric acid (1 N, 10 mL) was added to the reaction mixture, followed by extraction with ethyl acetate (2 × 20 mL). The mixture was washed with water (1 × 10 mL) and saturated brine (2 × 10 mL) in sequence, dried over anhydrous sodium sulfate, filtered, and concentrated under reduced pressure to give a crude product, which was then purified by column chromatography (petroleum ether/ethyl acetate) to give compound **17d** (140 mg, yield: 26%).

### Step 4: synthesis of methyl 6-chloro-3-(6-chloro-2,4-difluoro-3-methylphenyl)thieno[3,2-b]pyridine-2-carboxylate 17e

At room temperature, compound **17d** (100 mg, 0.32 mmol) and compound **1d** (140 mg, 0.48 mmol) were dissolved in toluene/water (2 mL/0.5 mL), and then tris(dibenzylideneacetone)dipalladium(0) (30 mg, 0.032 mmol), 2-dicyclohexylphosphino-2',6'-dimethoxybiphenyl (29 mg, 0.064 mmol), and potassium phosphate (216 mg, 0.96 mmol) were added. The mixture was stirred at 100 °C for 4 h in a nitrogen atmosphere. Ethyl acetate (60 mL) was added to the reaction system. The mixture was washed with water (1 × 10 mL) and saturated brine (2 × 10 mL) in sequence, dried over anhydrous sodium sulfate, filtered, and concentrated under reduced pressure to give a crude product, which was then purified by column chromatography (petroleum ether/ethyl acetate) to give compound **17e** (90 mg, yield: 72%).

MS(ESI) m/z = 388.2 [M+H]⁺.

### Step 5: synthesis of 6-chloro-3-(5-chloro-2,4-difluoro-3-methylphenyl)thieno[3,2-b]pyridine-2-carboxylic acid 17

At room temperature, compound **17e** (90 mg, 0.23 mmol) was dissolved in a mixed solvent of tetrahydrofuran (2 mL), methanol (1 mL), and water (1 mL), and lithium hydroxide (56 mg, 2.3 mmol) was added. The mixture was stirred for 1 h. The pH was adjusted to 4-5 with diluted hydrochloric acid (1 N). Ethyl acetate (40 mL) was added. The mixture was washed with water (1 × 10 mL) and saturated brine (2 × 10 mL) in sequence, dried over anhydrous sodium sulfate, filtered, and concentrated under reduced pressure to give a crude product of **17,** which was then separated and purified by preparative high performance liquid chromatography (column: Waters Xbridge, 30 × 250 mm; mobile phase A: 0.1% formic acid in water; mobile phase B: acetonitrile; flow rate: 30 mL/min, 18 min; gradient: 40%-95%; retention time: 11.99 min) to give the title compound **17** (37 mg, yield: 43%).

MS(ESI) m/z = 374.1 [M+H]⁺.

¹H-NMR(400 MHz, DMSO-*d*₆) δ 8.82 (s, 1H), 8.74 (s, 1H), 7.48 (s, 1H), 2.19 (s, 3H).

¹⁹F-NMR(376 MHz, DMSO-*d*₆) δ -111.12(s, 1F), δ =-112.74(s, 1F).

### Example 18

### 6-Chloro-3-{6-chloro-2,4-difluoro-3-[(trideuteriomethyl)oxy]phenyl}thieno[3,2-b]pyridine-2-carboxylic acid (18); 6-Chloro-3-{6-chloro-2,4-difluoro-3-[(trideuteriomethyl)oxy]phenyl}thieno[3,2-b]pyridine-2-carboxylic acid (18-P1); 6-Chloro-3-{6-chloro-2,4-difluoro-3-[(trideuteriomethyl)oxy]phenyl}thieno[3,2-b]pyridine-2-carboxylic acid (18-P2)

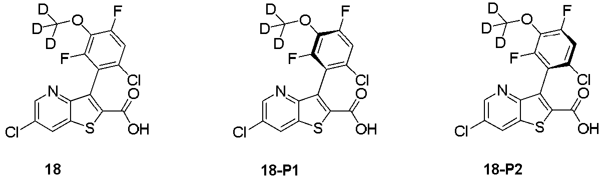

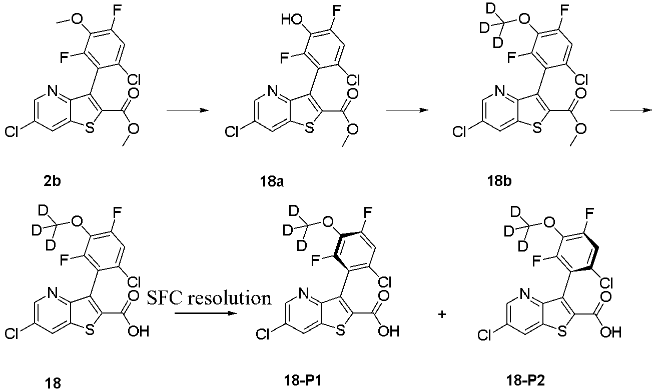

### Step 1: synthesis of methyl 6-chloro-3-(6-chloro-2,4-difluoro-3-hydroxyphenyl)thieno[3,2-b]pyridine-2-carboxylate 18a

Compound **2b** (5 g, 12.4 mmol) was dissolved in dichloromethane (10 mL) in an ice bath, and a solution of boron tribromide in dichloromethane (1 M, 50 mL) was slowly added dropwise. After the addition, the mixture was stirred at room temperature for 2 h. Methanol was slowly added to the system in an ice bath. After the addition, the mixture was stirred for 10 min and concentrated under reduced pressure to give a crude product of **18a** (4 g, yield: 82%), which was directly used in the next step.

MS(ESI) m/z = 390.3 [M+H]⁺.

### Step 2: synthesis of methyl 6-chloro-3-(6-chloro-2,4-difluoro-3-(methoxy-d3)phenyl)thieno[3,2-b]pyridine-2-carboxylate 18b

At room temperature, the crude product of **18a** (4 g, 10.3 mmol), deuterated iodomethane (0.71 mL, 11.3 mmol), and potassium carbonate (2.1 g, 15.4 mmol) were dissolved in N,N-dimethylformamide (5 mL). The mixture was stirred at 50 °C overnight. Water (30 mL) was added, followed by extraction with ethyl acetate (3 × 10 mL). The organic phases were combined, washed with saturated brine (2 × 10 mL), dried over anhydrous sodium sulfate, filtered, and concentrated under reduced pressure to give a crude product of **18b** (3.5 g, yield: 83%), which was directly used in the next step.

MS(ESI) m/z = 407.3 [M+H]⁺.

### Step 3: synthesis of 6-chloro-3-(6-chloro-2,4-difluoro-3-(methoxy-d3)phenyl)thieno[3,2-b]pyridine-2-carboxylic acid 18

At room temperature, compound **18b** (3.5 g, 8.6 mmol) was dissolved in a mixed solvent of tetrahydrofuran (6 mL), methanol (3 mL), and water (3 mL), and lithium hydroxide (2.1 g, 87 mmol) was added. The reaction mixture was stirred at room temperature for 2 h. Water (30 mL) was added. The pH was adjusted to 4-5 with hydrochloric acid (1 N), followed by extraction with ethyl acetate (2 × 30 mL). The organic phases were combined, washed with saturated brine (2 × 10 mL), dried over anhydrous sodium sulfate, filtered, and concentrated under reduced pressure to give a crude product, which was separated and purified by preparative high performance liquid chromatography (column: Waters Xbridge, 30 × 250 mm; mobile phase A: 0.1% formic acid in water; mobile phase B: acetonitrile; gradient: 35%-85%, 15 min; flow rate: 30 mL/min; peak time: 9.82 min) to give the title compound **18** (1.8 g, yield: 52%).

MS (ESI) m/z = 393.3 [M+H]⁺.

¹H-NMR (400 MHz, DMSO-*d₆*) δ 8.87 (d, 1H), 8.76 (d, 1H),7.67 (d, 1H).

¹⁹F-NMR (376 MHz, DMSO-*d₆*) δ -124.93 (s, 1F), -126.95 (s, 1F).

### Step 4: 6-chloro-3-{6-chloro-2,4-difluoro-3-[(trideuteriomethyl)oxy]phenyl}thieno[3,2-b]pyridine-2-carboxylic acid (18-P1); 6-chloro-3-16-chloro-2,4-difluoro-3-[(trideuteriomethyl)oxy]phenyl}thieno[3,2-b]pyridine-2-carboxylic acid (18-P2)

Compound **18** (92 mg) was resolved by SFC. Resolution method: column: ChiralPak AD, 250 × 30 mm I.D., 5 µm; mobile phase: A: CO₂; B: isopropanol [0.1% NH₃ (7 M in MeOH)]; gradient: B 20%; flow rate: 100 mL/min; back pressure: 100 bar; column temperature: 35 °C; wavelength: 214 nm; cycle time: about 5 min.
**18-P1,** retention time t = 2.819 min, 32 mg;
**18-P2,** retention time t = 2.626 min, 34 mg.

### Example 19

### 3-(6-Chloro-2,4-difluoro-3-methoxyphenyl)-6-fluorothieno[3,2-b]pyridine-2-carboxylic acid (19); 3-(6-Chloro-2,4-difluoro-3-methoxyphenyl)-6-fluorothieno[3,2-b]pyridine-2-carboxylic acid (19-P1); 3-(6-Chloro-2,4-difluoro-3-methoxyphenyl)-6-fluorothieno[3,2-b]pyridine-2-carboxylic acid (19-P2)

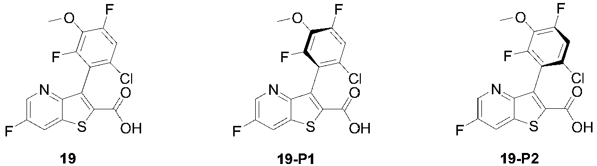

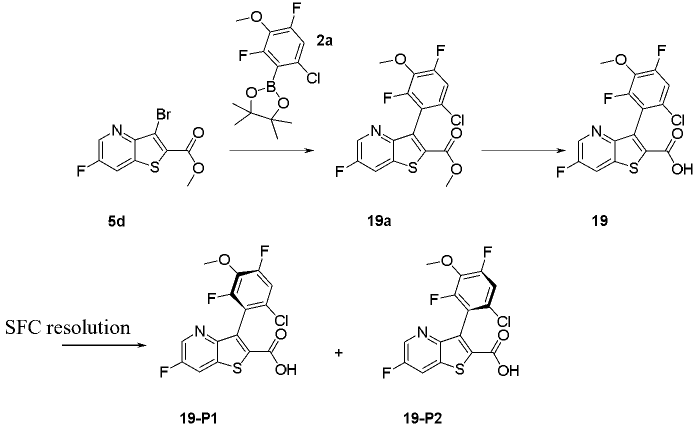

### Step 1: synthesis of 3-(6-chloro-2,4-difluoro-3-methoxyphenyl)-6-fluorothieno[3,2-b]pyridine-2-carboxylic acid (19)

3-(6-Chloro-2,4-difluoro-3-methoxyphenyl)-6-fluorothieno[3,2-b]pyridine-2-carboxylic acid (**19**) was prepared by using the synthesis method in Example **2** and replacing compound **1d** with compound **5d.**

Preparative high performance liquid chromatography separation and purification conditions for compound **19:** column: Waters Xbridge, 30 × 250 mm; mobile phase A: 0.1% formic acid in water; mobile phase B: acetonitrile; gradient: 35%-95%, 18 min; flow rate: 30 mL/min; peak time: 10.89 min.

MS (ESI) m/z = 374.2 [M+H]⁺.

¹H-NMR (400 MHz, DMSO-*d*₆) δ 8.77 (d, 1H), 8.65 (dd, 1H), 7.61 (dd, 1H), 3.95 (s, 3H).

¹⁹F-NMR (376 MHz, DMSO-*d*₆) δ -124.96 (d, 1F), -126.96 (d, 1F), -127.19 (s, 1F).

### Step 2: 3-(6-chloro-2,4-difluoro-3-methoxyphenyl)-6-fluorothieno[3,2-b]pyridine-2-carboxylic acid (19-P1); 3-(6-chloro-2,4-difluoro-3-methoxyphenyl)-6-fluorothieno[3,2-b]pyridine-2-carboxylic acid (19-P2)

The product **19** (520 mg) was resolved by SFC. Resolution method: column: ChiralPak IC, 250 × 30 mm I.D., 5 µm; mobile phase: A: CO₂; B: methanol [0.2% TFA]; gradient: B 20%; flow rate: 100 mL/min; back pressure: 100 bar; column temperature: 35 °C; wavelength: 210 nm; cycle time: about 2 min.
**19-P1,** retention time t = 2.409 min, 240 mg;
**19-P2,** retention time t = 1.993 min, 234 mg.

### Biological Evaluations

The following test examples further describe and illustrate the present disclosure, but these examples are not intended to limit the scope of the present disclosure.

### Test Example 1: Assay on Inhibitory Effect of Compounds on BCKDK Protein in HEK293 Cells

### 1. Experimental materials

| Name of reagent | Manufacturer | Cat. No. |
|---|---|---|
| DMEM (4.5 g/L) complete culture medium | Gibco | 11995040 |
| Phospho-BCKDH-E1α (Ser293) (E2V6B) rabbit mAb | CST | 40368S |
| CellTag 520S tain | Licor | 926-41094 |
| Intercept^{®} (TBS) blocking buffer | Licor | 927-60001 |
| IRDye^{®} 800CW goat anti-rabbit IgG secondary antibody | Licor | 926-32211 |
| Odyssey imager M | Licor | / |
| HEK293 cells | ATCC | CRL-1573 |

### 2. Test method

HEK293 cells were seeded in a 384-well black clear-bottom plate at 10,000 cells/well and incubated overnight. The cells were treated with different concentrations of compounds (100 µM maximum concentration, 3-fold dilution, 10 concentrations) at 37 °C with 5% carbon dioxide for 4 h. Pre-cooled 8% paraformaldehyde was added to the plate at 50 µL/well, and the cells were fixed for 1 h in the dark. The fixative solution was pipetted and discarded. A permeabilization solution was added at 50 µL/well, and the mixture was left to stand for 5 min each time. The process was repeated 6 times. A blocking buffer was then added at 50 µL/well, and the mixture was incubated on a shaker at room temperature for one hour. The primary antibody, Phospho-BCKDH-E1α (Ser293) (1000×), was added, followed by incubation at 4 °C for 24 h. After the permeabilization step was repeated again, the secondary antibodies, Cell Tag520 (500×) & IRDye 800CW (1000×), were added, followed by incubation at room temperature for 1 h in the dark. The permeabilization step was repeated once more, and the permeabilization solution was completely pipetted away at the last time. The signals were detected using the Odyssey imager M imaging system, data were processed using XL fit, and IC₅₀ was calculated.

### 3. Experimental results

The inhibition of the BCKDK protein in HEK293 cells by the compounds of the present disclosure was determined by the above assay. The IC₅₀ values obtained are shown in Table 1.

**Table 1. IC₅₀ (µM) of compounds of the present disclosure against HEK293A**

| Example No. | IC₅₀ (µM) |
|---|---|
| **1** | 5.66 |
| **2-P1** | 0.96 |
| **3** | 4.72 |
| **4** | 2.61 |
| **5** | 9.66 |
| **6** | 2.83 |
| **7** | 1.36 |
| **8** | 4.36 |
| **9** | 5.92 |
| **10** | 3.57 |
| **11-P1** | 0.56 |
| **15** | 4.13 |
| **18-P1** | 0.89 |
| **19-P1** | 1.56 |
| **PF-07328948** | 1.69 |

**PF-07328948** was the compound of Example 9 in the patent WO2023100061A1, with the structure being

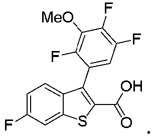

### Test Example 2: Inhibitory Activity of Compounds Against Human CYP450 (CYP1A2, CYP2C9, CYP2C19, CYP2D6, and CYP3A)

### 1. Equipment, materials, and reagents

### 1) Human liver microsome (HLM)

Human liver microsomes (source: BioIVT) were stored in a freezer at -80 °C. Before use, the human liver microsomes were taken out from the freezer and pre-heated and thawed in a 37 °C water bath, and then placed on ice for later use.

### 2) Substrate

Details of the preparation of a substrate mixture solution are as follows: The substrate mixture solution was composed of 50% of purified water and 50% of organic solvent, and the prepared substrate mixture solution was stored in a freezer at -20 °C, which was warmed to room temperature and then vortexed for 30 s before use.

Substrate mixture solutions of CYP1A2, CYP2C9, CYP2C19, CYP2D6, and CYP3A

| CYP Enzyme | Substrate | MW (g/mol) | Stock solution concentration (mM) | Addition volume (µL) | Total volume (µL) | Working solution concentration (mM) | Final concentration (µM) |
|---|---|---|---|---|---|---|---|
| 1A2 | Phenacetin | 179.2 | 30 (dissolved in acetonitrile) | 50 | 250 | 6 | 30 |
| 2C9 | Diclofenac sodium | 318.1 | 10 (dissolved in water) | 50 | | 2 | 10 |
| 2C19 | *S-*mephenytoin | 218.2 | 35 (dissolved in acetonitrile) | 50 | | 7 | 35 |
| 2D6 | Bufuralol hydrochloride | 297.8 | 5 (dissolved in water) | 50 | | 1 | 5 |
| 3A | Midazolam | 325.8 | 3 (dissolved in DMSO) | 50 | | 0.6 | 3 |

### 3) Phosphate buffer (100 mmol/L, pH 7.4)

Disodium hydrogen phosphate (analytically pure) and potassium dihydrogen phosphate (analytically pure) were purchased from local suppliers. Preparation of solution A: 7.098 g of disodium hydrogen phosphate was accurately weighed out, and 500 mL of purified water was added. The mixture was subjected to ultrasonic treatment. Preparation of solution B: 3.400 g of potassium dihydrogen phosphate was accurately weighed out, and 250 mL of purified water was added. The mixture was subjected to ultrasonic treatment. The solution B was added to the solution A, and the final pH was 7.4.

### 4) 10 mmol/L NADPH solution

NADPH (MW: 833.4 g/mol) was purchased from MCE and dissolved at 8.334 mg/mL in a phosphate buffer.

### 2. Experimental process

### 1) Preparation of incubation solution

The incubation system for the mixed solution of the substrate and human liver microsome is as follows:

| Buffer | Working solution concentration | Volume | Final concentration |
|---|---|---|---|
| Liver microsome | 20 mg/mL | 2 µL | 0.2 mg/mL |
| Phosphate buffer | 100 mM | 176 µL | 100 mM |
| Substrate | - | 1 µL | - |

### 2) Compound dilution

15 µL of a 10 mmol/L DMSO solution of the test compound was added to a deep-well plate containing sequentially labeled compounds. The dilution steps are as follows:

| Dilution of test compounds | | | | |
|---|---|---|---|---|
| Stock solution/Position | Volume of compound (µL) | Volume of DMSO (µL) | Working solution concentration (mM) | Final concentration (µM) |
| A1 | 15 µL of 10 mM stock solution | 10 | 6 | 30 |
| B1 | 9 µL A1 | 18 | 2 | 10 |
| C1 | 9 µL B1 | 21 | 0.6 | 3 |
| D1 | 9 µL C1 | 18 | 0.2 | 1 |
| E1 | 9 µL D1 | 21 | 0.06 | 0.3 |
| F1 | 9 µL E1 | 18 | 0.02 | 0.1 |

### 3) Incubation

Each well of a 96-well deep-well plate contained 179 µL of a mixed solution of the substrate and human liver microsome phosphate buffer, and 1 µL of the test compound or a blank solution. The incubation plate was placed in a 37 °C water bath and pre-incubated for 5 min, and then 20 µL of 10 mM NADPH was added to initiate the reaction. After the addition of NADPH solution, the incubation plate was incubated for another 5 min at 37 °C.

### 4) Quenching of reaction

The reaction was quenched by adding 300 µL of an acetonitrile solution containing 3% formic acid, 200 nM tolbutamide, 200 nM alprazolam, and 200 nM labetalol. The mixture was then centrifuged in a centrifuge at 3220 g for 50 min, and 200 µL of the supernatant was taken for LC/MS/MS analysis.

### 3. Data processing

The peak areas of all samples and the peak area of an internal standard were calculated automatically and imported into Excel software.

The percentage of residual activity was calculated according to the following formula:$Area ratio = peak {area}_{test compound} / peak {area}_{internal standard}$$Percentage of residual activity \left(%\right) = area {ratio}_{test compound} / area {ratio}_{blank} \times 100$

The IC₅₀ values (the concentration of the test compound at which 50% inhibition was achieved) were calculated using Excel XLfit 5.3.1.3.

**Table 2. Inhibitory activity IC₅₀ (µM) of compounds of the present disclosure against CYP450**

| Test compound | CYP1A2 | CYP2C9 | CYP2C19 | CYP2D6 | CYP3A |
|---|---|---|---|---|---|
| **19-P1** | >30 | >30 | >30 | >30 | >30 |
| **PF-07328948** | >30 | 1.09 | >30 | >30 | >30 |
| Reference compound **1** | 5.0 | 2.32 | 15.76 | >30 | >30 |

Reference compound **1** was the compound of Example **16** in the patent WO2023100061A1.

According to the results, compound **19-P1** did not inhibit CYP1A2, CYP2C9, CYP2C19, CYP2D6, and CYP3A (IC₅₀ > 30 µM), **PF-07328948** showed strong inhibition on CYP2C9 (IC₅₀ = 1.09 µM), and reference compound **1** showed relatively strong inhibition on CYP1A2 (IC₅₀ = 5.0 µM), CYP2C9 (IC₅₀ > 2.32 µM), and CYP2C19 (IC₅₀ = 15.76 µM).

### Test Example 3: Stability Test of Compounds in Hepatocytes

### 1. Experimental steps

1) The test compound was dissolved in DMSO to prepare a high-concentration (10 mM) stock solution. Before use, the stock solution was diluted with DMSO to prepare a 100 µM working solution. The final concentration of the test compound was 1 µM.
2) A tube of cryopreserved hepatocytes (source of rat, dog, and human hepatocytes: BioIVT; source of monkey hepatocytes: RILD) was taken. It was ensured that the hepatocytes were still in a low-temperature frozen state before thawing. The hepatocytes were quickly placed in a 37 °C water bath, gently shaken until all ice crystals were dispersed, sprayed with 70% ethanol, and then transferred to a biosafety cabinet.
3) The contents of small tubes of hepatocytes from different species were poured into a centrifuge tube containing 50 mL of thawing culture medium and centrifuged at 100 g for 10 min. After centrifugation, the thawing culture medium was pipetted off, and a sufficient amount of incubation culture medium was added to obtain a cell suspension with a cell density of about 1.0 × 10⁶ cells/mL.
4) Hepatocytes were counted using Cellometer Vision, and the viable cell density was determined. The viability of hepatocytes must be greater than 75%. The hepatocyte suspension was diluted with the incubation culture medium to a viable cell density of 0.5 × 10⁶ cells/mL.
5) 198 µL of the viable cell suspension was transferred to a 96-well deep-well plate, and the deep-well plate was placed on a vortexer and pre-heated in an incubator for 10 min. In the experiment, the incubation was performed in duplicate.
6) 2 µL of the 100 µM test compound was added to each well to start a reaction, and the deep-well plate was placed back on the vortexer in the incubator.
7) The samples were incubated. At 0 min, 15 min, 30 min, 60 min, 90 min, and 120 min, 25 µL of the suspension was taken, and 150 µL of acetonitrile containing an internal standard was added to stop the reaction. After 10 min of vortexing, the mixture was centrifuged at 3220 g at 4 °C for 45 min. 100 µL of the supernatant was transferred to a sample injection plate, and 100 µL of purified water was added. After thorough mixing, the mixture was analyzed by UPLC-MS/MS.

### 2. Data analysis

All data calculations were performed through Microsoft Excel software. The peak areas were determined through an extracted ion chromatogram. Through linear fitting of the natural logarithm of the parent drug elimination percentage versus time, the *in vitro* clearance rate of the parent drug was determined.

The *in vitro* clearance rate (µL/min/10⁶ cells) was calculated using the following formula: *In vitro* CLᵢₙₜ = kV/N,
where V = the incubation volume per well (0.2 mL);
N = the number of cells per well (0.1 × 10⁶ cells).

**Table 3. Metabolic clearance rates CLᵢₙₜ (µL/min/10⁶ cells) of compounds in hepatocytes**

| Test compound | Rat | Dog | Monkey | Human |
|---|---|---|---|---|
| **19-P1** | 20.18 | 0 | 0 | 2.38 |
| **PF-07328948** | 29.05 | 7.04 | 28.67 | 15.03 |
| Reference compound **1** | 29.23 | 18.73 | 43.98 | 15.35 |

According to the results, compound **19-P1** exhibited significantly greater metabolic stability in dog, monkey, and human hepatocytes than **PF-07328948** and reference compound **1.**

### Test Example 4: In Vivo Pharmacokinetic Experiment in Rats and Dogs

### 1. In vivo pharmacokinetic experiment in rats

SD rats (source: Zhejiang Vital River Laboratory Animal Technology Co., Ltd.) were used as test animals. The plasma concentrations of the test compound at different time points after intragastric administration of the test compound to rats were determined using an LC/MS/MS method. The pharmacokinetic behavior of the test compound in rats was studied, and its pharmacokinetic profile was evaluated.

Test animals: two healthy male rats aged 6-8 weeks (200-300 g) per group.

Compound solution preparation: A certain amount of the compound was weighed out and dissolved in a vehicle (100% normal saline and 1 mol/L hydrochloric acid and sodium hydroxide, allowing the final solution to be adjusted to pH 7) to form a 2 mg/mL colorless, clear solution.

Dosing: Rats were fasted overnight and then intragastrically dosed. The test compound was administered at a dose of 20 mg/kg.

Procedure: Rats were intragastrically administered with the test compound. At 0.25 h, 0.5 h, 1 h, 2 h, 4 h, 6 h, 8 h, and 24 h after administration, blood samples (about 0.2 mL) were collected via jugular catheterization, placed into test tubes containing EDTA-K2, and centrifuged at around 4 °C at 4000 g per minute for 5 min to separate plasma, and the plasma was stored at -75±15 °C.

Determination of the content of the compound to be tested in rat plasma after the test compound was orally administered: 50 µL of the rat plasma at each time point after administration was vortexed with 5 µL of a blank solution and 200 µL of a solution of dexamethasone (internal standard) in acetonitrile for 30 s, and the mixture was centrifuged for 15 min (3900 rpm). 100 µL of the supernatant of the plasma sample was mixed well with 200 µL of ultrapure water, and then 5 µL of the dilution was taken using a syringe for LC/MS/MS analysis.

**Table 4. Pharmacokinetic parameters of compounds after oral administration to rats (20 mpk)**

| Test compound | T_{1/2} (h) | Cₘₐₓ (ng/mL) | AUCₗₐₛₜ (h*ng/mL) |
|---|---|---|---|
| **19-P1** | NA | 77900 | 1297444 |
| **PF-07328948** | 4.11 | 51250 | 223769 |

| | | | |
|---|---|---|---|
| Note: All the shown values were the mean of two animals. | | | |

According to the results, compound **19-P1** exhibited significantly greater oral exposure in rats than **PF-07328948.**

### 2. In vivo pharmacokinetic experiment in dogs

Beagles (source: Beijing Marshall Biotechnology Co., Ltd.) were used as test animals. The plasma concentrations of the test compound at different time points after intragastric administration of the test compound to beagles were determined using an LC/MS/MS method. The pharmacokinetic behavior of the test compound in beagles was studied, and its pharmacokinetic profile was evaluated.

Test animals: two healthy male beagles aged 8 months to 3 years (6.0-13.0 kg) per group. Compound solution preparation: A certain amount of the compound was weighed out and dissolved in a vehicle (100% normal saline and 1 mol/L hydrochloric acid and sodium hydroxide, allowing the final solution to be adjusted to pH 7) to form a 0.6 mg/mL colorless, clear solution.

Dosing: Beagles were fasted overnight and then intragastrically dosed. The test compound was administered at a dose of 3 mg/kg.

Procedure: Beagles were intragastrically administered with the test compound. Before administration and at 0.25 h, 0.5 h, 1 h, 2 h, 4 h, 6 h, 8 h, and 24 h after administration, blood samples (about 0.6 mL) were collected via peripheral venipuncture, placed into test tubes containing EDTA-K2, and centrifuged at around 4 °C at 2000 g per minute for 10 min to separate plasma, and the plasma was stored at -75±15 °C.

Determination of the content of the compound to be tested in beagle plasma after the test compound was orally administered: 50 µL of the beagle plasma at each time point after administration was vortexed with 5 µL of a blank solution and 200 µL of a solution of dexamethasone (internal standard) in acetonitrile for 30 s, and the mixture was centrifuged for 15 min (3900 rpm). 100 µL of the supernatant of the plasma sample was mixed well with 200 µL of ultrapure water, and then 5 µL of the dilution was taken using a syringe for LC/MS/MS analysis.

**Table 5. Pharmacokinetic parameters of compounds after oral administration to dogs (3 mpk)**

| Test compound | T_{1/2} (h) | Cₘₐₓ (ng/mL) | AUCₗₐₛₜ (h*ng/mL) |
|---|---|---|---|
| **19-P1** | 5.77 | 18250 | 102182 |
| **PF-07328948** | 5.42 | 13900 | 20741 |

| | | | |
|---|---|---|---|
| Note: All the shown values were the mean of two animals. | | | |

According to the results, compound **19-P1** exhibited significantly greater oral exposure in dogs than **PF-07328948.**

## Claims

1. A compound represented by formula (I) or a pharmaceutically acceptable salt thereof, 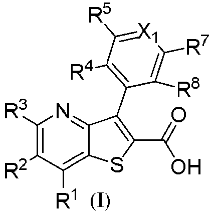
wherein R¹, R², and R³ are each independently selected from the group consisting of hydrogen, fluorine, chlorine, and methyl;
R⁴ is selected from the group consisting of fluorine and chlorine;
R⁵ is selected from the group consisting of C₁₋₄ alkyl, C₁₋₄ haloalkyl, 3- to 4-membered cycloalkyl, and -L₁-R⁹, wherein L₁ is selected from the group consisting of sulfur and oxygen, and R⁹ is selected from the group consisting of C₁₋₄ alkyl, C₁₋₄ haloalkyl, deuterated C₁₋₄ alkyl, and 3- to 4-membered cycloalkyl;
X₁ is selected from the group consisting of nitrogen and CR⁶, wherein R⁶ is selected from the group consisting of hydrogen, fluorine, and chlorine;
R⁷ and R⁸ are each independently selected from the group consisting of hydrogen, fluorine, and chlorine.

2. The compound represented by formula (I) or the pharmaceutically acceptable salt thereof according to claim 1, wherein X₁ is nitrogen.

3. The compound represented by formula (I) or the pharmaceutically acceptable salt thereof according to claim 1, being a compound represented by formula (II) or a pharmaceutically acceptable salt thereof, 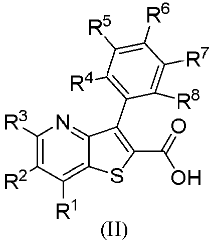 wherein R¹, R², R³, R⁴, R⁵, R⁶, R⁷, and R⁸ are each as defined in claim 1.

4. The compound or the pharmaceutically acceptable salt thereof according to any one of claims 1 to 3, wherein R⁵ is selected from the group consisting of C₁₋₄ alkyl and C₁₋₄ haloalkyl; preferably, R⁵ is selected from the group consisting of methyl, ethyl, and isopropyl; most preferably, R⁵ is selected from the group consisting of methyl and ethyl.

5. The compound or the pharmaceutically acceptable salt thereof according to any one of claims 1 to 3, wherein R⁵ is selected from the group consisting of 3- to 4-membered cycloalkyl; preferably, R⁵ is cyclopropyl.

6. The compound or the pharmaceutically acceptable salt thereof according to any one of claims 1 to 3, being a compound represented by formula (II-1) or a pharmaceutically acceptable salt thereof, 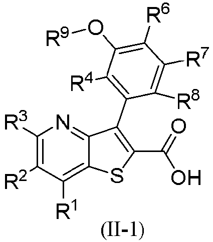 wherein R¹, R², R³, R⁴, R⁶, R⁷, R⁸, and R⁹ are each as defined in claim 1.

7. The compound or the pharmaceutically acceptable salt thereof according to claim 6, wherein R⁹ is selected from the group consisting of C₁₋₄ alkyl, C₁₋₄ haloalkyl, and deuterated C₁₋₄ alkyl; preferably, R⁹ is selected from the group consisting of methyl, ethyl, difluoromethyl, trifluoromethyl, and deuterated methyl; most preferably, R⁹ is selected from the group consisting of methyl, difluoromethyl, trifluoromethyl, and deuterated methyl.

8. The compound or the pharmaceutically acceptable salt thereof according to any one of claims 1 to 3, being a compound represented by formula (II-2) or a pharmaceutically acceptable salt thereof, 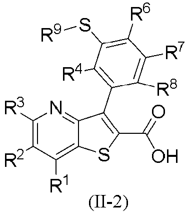 wherein R¹, R², R³, R⁴, R⁶, R⁷, R⁸, and R⁹ are each as defined in claim 1.

9. The compound or the pharmaceutically acceptable salt thereof according to claim 8, wherein R⁹ is selected from the group consisting of C₁₋₄ alkyl, C₁₋₄ haloalkyl, and deuterated C₁₋₄ alkyl; preferably, R⁹ is selected from the group consisting of methyl, ethyl, difluoromethyl, trifluoromethyl, and deuterated methyl; most preferably, R⁹ is methyl.

10. The compound or the pharmaceutically acceptable salt thereof according to any one of claims 1 to 9, wherein at least one of R¹, R², and R³ is selected from the group consisting of fluorine and chlorine.

11. The compound or the pharmaceutically acceptable salt thereof according to any one of claims 1 to 10, wherein R¹ and R³ are each independently selected from the group consisting of hydrogen, fluorine, and chlorine; preferably, one of R¹ and R³ is hydrogen, and the other is selected from the group consisting of hydrogen, fluorine, and chlorine; most preferably, R¹ and R³ are each independently hydrogen.

12. The compound or the pharmaceutically acceptable salt thereof according to any one of claims 1 to 11, wherein R² is fluorine or chlorine; preferably, R² is fluorine; preferably, R² is chlorine.

13. The compound or the pharmaceutically acceptable salt thereof according to any one of claims 1 to 12, wherein R¹ and R³ are each independently hydrogen; R² is fluorine or chlorine.

14. The compound or the pharmaceutically acceptable salt thereof according to any one of claims 1 to 13, wherein R⁴ is selected from the group consisting of fluorine and chlorine; preferably, R⁴ is fluorine.

15. The compound or the pharmaceutically acceptable salt thereof according to any one of claims 1 to 14, wherein R¹ and R³ are each independently hydrogen, R² is fluorine or chlorine, and R⁴ is fluorine.

16. The compound or the pharmaceutically acceptable salt thereof according to any one of claims 1 to 15, wherein at least one of R⁶, R⁷, and R⁸ is selected from the group consisting of fluorine and chlorine; preferably, at least two of R⁶, R⁷, and R⁸ are selected from the group consisting of fluorine and chlorine.

17. The compound or the pharmaceutically acceptable salt thereof according to any one of claims 1 to 16, wherein R⁶ is selected from the group consisting of fluorine and chlorine.

18. The compound or the pharmaceutically acceptable salt thereof according to any one of claims 1 to 17, wherein at least one of R⁶, R⁷, and R⁸ is chlorine.

19. The compound or the pharmaceutically acceptable salt thereof according to any one of claims 1 to 18, wherein R⁷ is selected from the group consisting of fluorine and chlorine, and R⁸ is hydrogen; preferably, R⁷ is fluorine, and R⁸ is hydrogen.

20. The compound or the pharmaceutically acceptable salt thereof according to any one of claims 1 to 19, wherein R⁸ is selected from the group consisting of fluorine and chlorine, and R⁷ is hydrogen.

21. The compound or the pharmaceutically acceptable salt thereof according to any one of claims 1 to 20, wherein R¹ and R³ are each independently hydrogen; R² is fluorine or chlorine; R⁴ is fluorine; R⁶ is selected from the group consisting of fluorine and chlorine; R⁷ is selected from the group consisting of fluorine and chlorine, and R⁸ is hydrogen; or R⁸ is selected from the group consisting of fluorine and chlorine, and R⁷ is hydrogen; and at least one of R⁶, R⁷, and R⁸ is chlorine.

22. The compound or the pharmaceutically acceptable salt thereof according to any one of claims 1 to 21, being a compound represented by formula (II-A) or formula (II-B) or a pharmaceutically acceptable salt thereof, preferably a compound represented by formula (II-B) or a pharmaceutically acceptable salt thereof, 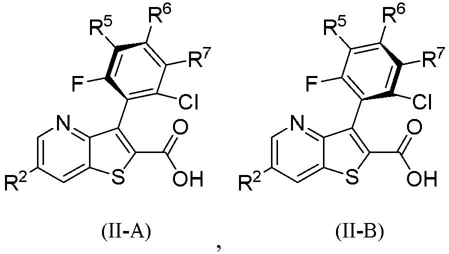 wherein R², R⁵, R⁶, and R⁷ are each as defined in claims 1 to 21; preferably, R² is chlorine; or preferably, R² is fluorine.

23. The compound or the pharmaceutically acceptable salt thereof according to claim 22, wherein R⁵ is selected from the group consisting of methyl and ethyl.

24. The compound or the pharmaceutically acceptable salt thereof according to claim 22, wherein R⁵ is selected from the group consisting of -L₁-R⁹, wherein L₁ is oxygen, and R⁹ is selected from the group consisting of methyl, ethyl, difluoromethyl, trifluoromethyl, and deuterated methyl; preferably, R⁹ is selected from the group consisting of methyl and deuterated methyl; or preferably, R⁹ is selected from the group consisting of difluoromethyl and trifluoromethyl.

25. The compound or the pharmaceutically acceptable salt thereof according to claim 22, wherein R⁵ is selected from the group consisting of -L₁-R⁹, wherein L₁ is sulfur, and R⁹ is selected from the group consisting of methyl, ethyl, difluoromethyl, trifluoromethyl, and deuterated methyl; preferably, R⁵ is selected from the group consisting of -L₁-R⁹, wherein L₁ is sulfur, and R⁹ is methyl or deuterated methyl.

26. The compound or the pharmaceutically acceptable salt thereof according to claim 22, wherein R⁶ is fluorine or chlorine, and R⁷ is hydrogen; preferably, R⁶ is fluorine, and R⁷ is hydrogen.

27. The compound or the pharmaceutically acceptable salt thereof according to any one of claims 1 to 16, 18, 19, and 21, being a compound represented by formula (II-C) or a pharmaceutically acceptable salt thereof, 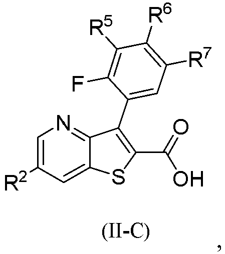 wherein R², R⁵, R⁶, and R⁷ are each as defined in claims 1 to 16, 18, 19, and 21; preferably, R² is chlorine; or preferably, R² is fluorine.

28. The compound or the pharmaceutically acceptable salt thereof according to claim 27, wherein R⁵ is selected from the group consisting of methyl and ethyl.

29. The compound or the pharmaceutically acceptable salt thereof according to claim 27, wherein R⁵ is selected from the group consisting of -L₁-R⁹, wherein L₁ is oxygen; R⁹ is selected from the group consisting of methyl, ethyl, difluoromethyl, trifluoromethyl, and deuterated methyl; preferably, R⁹ is selected from the group consisting of methyl and deuterated methyl, or preferably, R⁹ is selected from the group consisting of difluoromethyl and trifluoromethyl.

30. The compound or the pharmaceutically acceptable salt thereof according to claim 27, wherein R⁵ is selected from the group consisting of -L₁-R⁹, wherein L₁ is sulfur; R⁹ is selected from the group consisting of methyl, ethyl, difluoromethyl, trifluoromethyl, and deuterated methyl; preferably, R⁹ is methyl or deuterated methyl.

31. The compound or the pharmaceutically acceptable salt thereof according to claim 27, wherein at least one of R⁶ and R⁷ is selected from the group consisting of fluorine and chlorine; preferably, R⁶ is selected from the group consisting of fluorine and chlorine; most preferably, R⁶ is selected from the group consisting of fluorine, and R⁷ is selected from the group consisting of fluorine and chlorine; or R⁶ is selected from the group consisting of chlorine, and R⁷ is selected from the group consisting of fluorine and chlorine.

32. The compound or the pharmaceutically acceptable salt thereof according to any one of claims 1 to 31, being selected from the group consisting of the following compounds or pharmaceutically acceptable salts thereof: 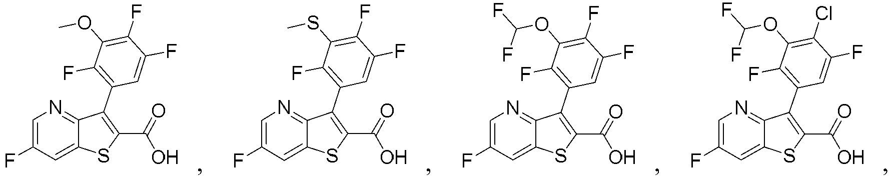 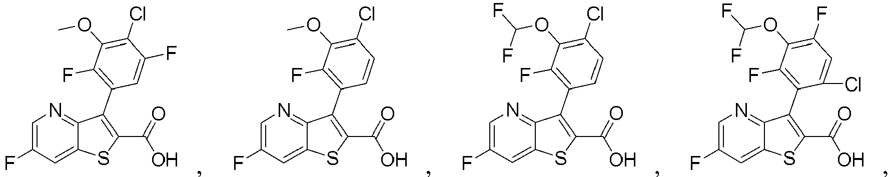 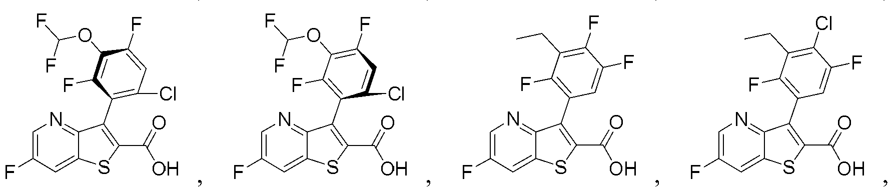 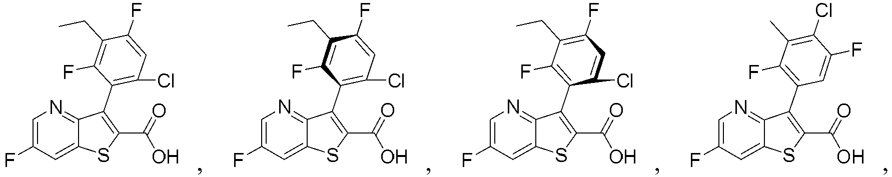 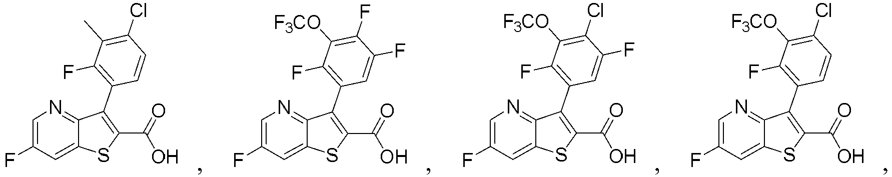 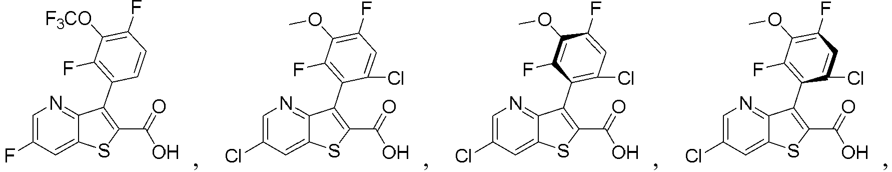 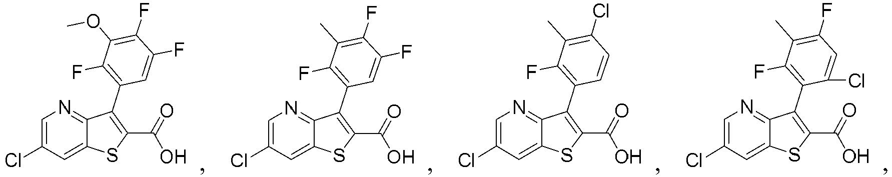 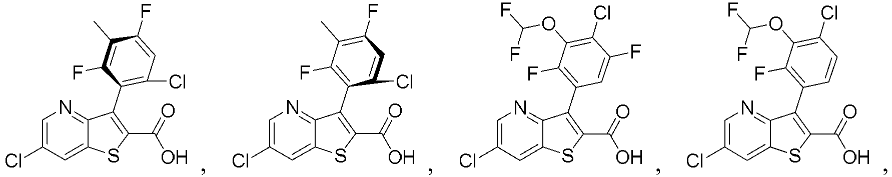 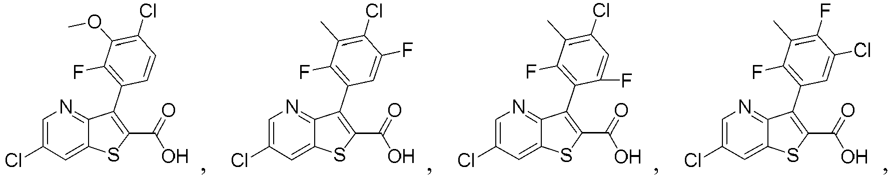 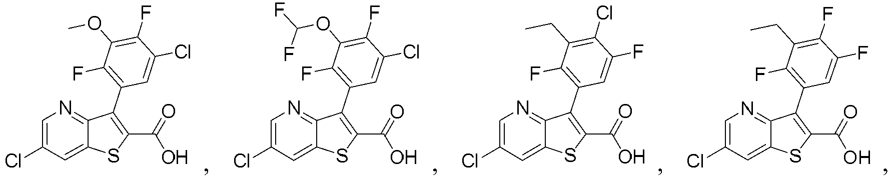 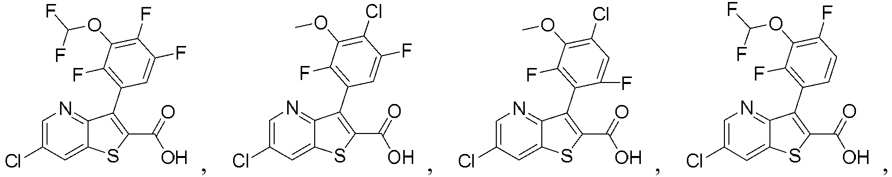 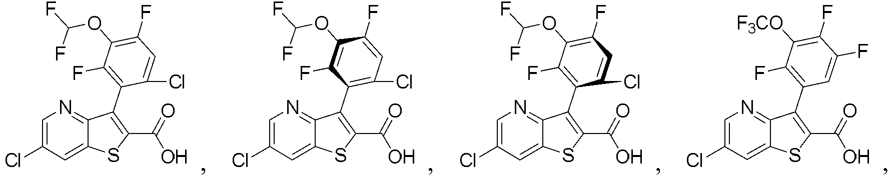 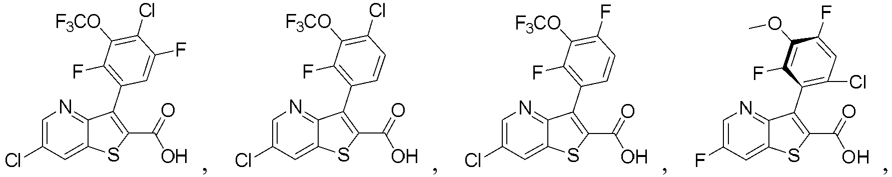 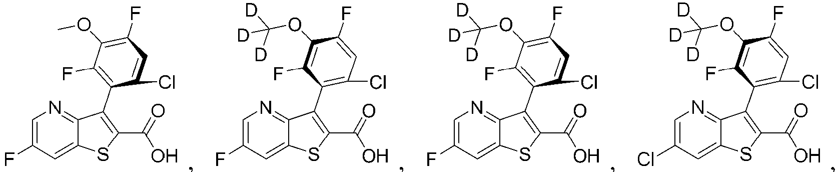 and

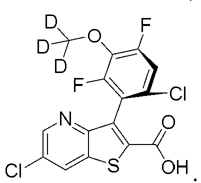

33. An isotopically substituted form of the compound or the pharmaceutically acceptable salt thereof according to any one of claims 1 to 32, wherein preferably, the isotopically substituted form is a deuterated form.

34. A pharmaceutical composition, comprising the compound or the pharmaceutically acceptable salt thereof according to any one of claims 1 to 32, or the isotopically substituted form according to claim 33, and one or more pharmaceutically acceptable excipients.

35. Use of the compound or the pharmaceutically acceptable salt thereof according to any one of claims 1 to 32, or the isotopically substituted form according to claim 33, or the pharmaceutical composition according to claim 34 in the manufacture of a medicament for preventing and/or treating a BCKDK-related disease, wherein preferably, the BCKDK-related disease is selected from the group consisting of a glucose metabolism disorder or an abnormal blood glucose disease, a heart disease, and a kidney disease; more preferably, the glucose metabolism disorder or the abnormal blood glucose disease is diabetes, and the heart disease is heart failure.

36. Use of the compound or the pharmaceutically acceptable salt thereof according to any one of claims 1 to 32, or the isotopically substituted form according to claim 33, or the pharmaceutical composition according to claim 34 in the manufacture of a medicament for preventing and/or treating a disease, wherein the disease is selected from the group consisting of a glucose metabolism disorder or an abnormal blood glucose disease, a heart disease, and a kidney disease; preferably, the glucose metabolism disorder or the abnormal blood glucose disease is diabetes, and the heart disease is heart failure.

37. A preparation method for the compound or the pharmaceutically acceptable salt thereof according to any one of claims 1 to 32, comprising a step of removing a protecting group PG from a compound represented by formula (I-a) or a pharmaceutically acceptable salt thereof under an alkaline condition, and further comprising a step of adjusting the pH to acidity, 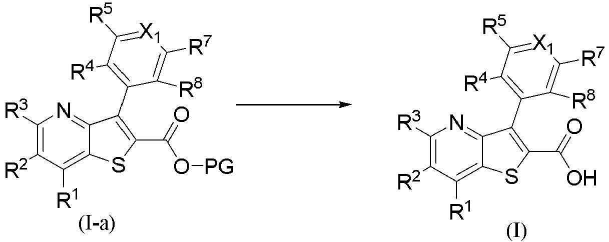 wherein PG is a carboxyl protecting group; R¹, R², R³, R⁴, R⁵, X₁, R⁷, and R⁸ are each as defined in claims 1 to 32; preferably, PG is C₁₋₆ alkyl.

38. A compound represented by formula (I-a) or a pharmaceutically acceptable salt thereof, 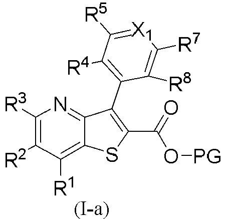 wherein PG is a carboxyl protecting group; R¹, R², R³, R⁴, R⁵, X₁, R⁷, and R⁸ are each as defined in claims 1 to 32; preferably, PG is C₁₋₆ alkyl.
